(19) Europäisches Patentamt
European Patent Office
Office européen des brevets

(11) Veröffentlichungsnummer: **0 150 461**
**B1**

(12) # EUROPÄISCHE PATENTSCHRIFT

(45) Veröffentlichungstag der Patentschrift: **30.03.88**

(51) Int. Cl.⁴: **C 07 C 177/00**, C 07 F 7/18, C 07 D 309/12, A 61 K 31/557

(21) Anmeldenummer: **84116000.5**

(22) Anmeldetag: **20.12.84**

(54) **Schwefelhaltige 6-Ketoprostaglandine.**

(30) Priorität: **18.01.84 DE 3401542**

(43) Veröffentlichungstag der Anmeldung: **07.08.85 Patentblatt 85/32**

(45) Bekanntmachung des Hinweises auf die Patenterteilung: **30.03.88 Patentblatt 88/13**

(84) Benannte Vertragsstaaten: **AT BE CH DE FR GB IT LI NL SE**

(56) Entgegenhaltungen:
**EP - A - 0 019 069**
**DE - B - 2 513 371**
**US - A - 4 205 178**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Irmscher, Klaus, Dr., Reuterallee 10, D-6100 Darmstadt-Eberstadt (DE)**
Erfinder: **Radunz, Hans-Eckart Dr., Am Klingenteich 5, D-6109 Mühltal-4 (DE)**
Erfinder: **Schulze, Ernst Dr., Auf der Hardt 73, D-6100 Darmstadt (DE)**
Erfinder: **Riefling, Bernhard Dr., Zöllerstrasse 28, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft 6-Ketoprostaglandine der allgemeinen Formel I

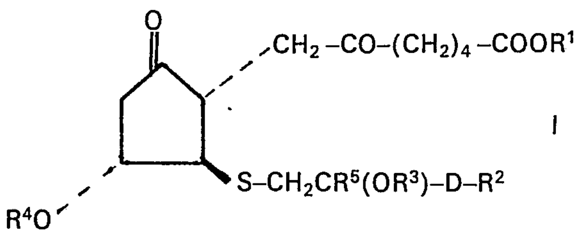

I

worin

D eine Bindung, Alkylen mit 1–3 C-Atomen, cis-Alkenylen mit 2–5 C-Atomen oder Alkinylen mit 2–5 C-Atomen,

$R^1$ H, Alkyl mit 1–4 C-Atomen, Aryl mit 6–12 C-Atomen oder $-C_6H_4NHCOC_6H_5$,

$R^2$ Alkyl mit 1–7 C-Atomen, durch Halogen substituiertes Alkyl mit 1–7 C-Atomen, Cycloalkyl mit 5–6 C-Atomen, durch Alkyl mit 1–4 C-Atomen substituiertes Cycloalkyl mit 5–6 C-Atomen, Phenyl, durch F, Cl, Br, Alkyl mit 1–4 C-Atomen, OH, $OCH_3$ oder $CF_3$ substituiertes Phenyl, Pyridyl, Naphthyl, Thienyl, oder, falls D Alkylen mit 1–3 C-Atomen bedeutet, auch Alkoxy mit 1–4 C-Atomen, Alkylthio mit 1–4 C-Atomen, Phenoxy oder durch F, Cl, Br, Alkyl mit 1–4 C-Atomen, OH, $OCH_3$ oder $CF_3$ substituiertes Phenoxy,

$R^3$ und $R^4$ jeweils H, Alkyl mit 1–7 C-Atomen, Tetrahydropyran-2-yl, Trialkylsilyl mit insgesamt 3–12 C-Atomen, Aryl-dialkylsilyl mit insgesamt 8–18 C-Atomen, Alkoxymethyl mit 2–5 C-Atomen, Aryloxymethyl mit 7–11 C-Atomen oder Acyl mit 1–10 C-Atomen und

$R^5$ H oder Alkyl mit 1–3 C-Atomen bedeuten und

....... anzeigt, dass diese Bindung α-ständig ist,

anzeigt, dass diese Bindung β-ständig ist,

sowie, falls $R^1$ =H ist, deren Salze.

Seit einiger Zeit haben Verbindungen pharmakologisches und medizinisches Interesse gewonnen, welche unter dem Begriff «Prostacycline» zusammengefasst werden können. Prostacyclin oder $PGI_2$, ein kürzlich isolierter Naturstoff aus der Familie der Prostaglandine, zeichnet sich durch ausgeprägte thrombozytenaggregationshemmende Eigenschaften aus (The Lancet 1977, 18). Die physiologischen Wirkungen von Prostaglandinen und Prostacyclinen sind jedoch sowohl in vitro als auch am Säugetierorganismus von kurzer Dauer, da sie schnell in pharmakologisch unwirksame Metabolite umgewandelt werden. Darüberhinaus ist es nachteilig, dass diese Verbindungen neben der gewünschten physiologischen Wirkung gleichzeitig eine Reihe von unerwünschten physiologischen Nebenwirkungen besitzen, die ihre Verwendung als Arzneimittel stark einschränken. Derartige, den Verbindungen der Formel I ähnlich Substanzen sind z.B. aus der DE-A-2 513 371, der US-A-4 205 178 und der EP-A-19 060 bekannt.

Es bestand deshalb die Aufgabe, neue Verbindungen mit differenzierten pharmakologischen Wirkungen aufzufinden, die zur Herstellung von Arzneimitteln verwendet werden können. Diese Aufgabe wurde durch die Bereitstellung der Verbindungen der allgemeinen Formel I gelöst.

Es wurde gefunden, dass die Verbindungen der allgemeinen Formel I und ihre physiologisch unbedenklichen Salze bei guter Verträglichkeit wertvolle pharmakologische Eigenschaften besitzen. So zeigen sich beispielsweise Wirkungen auf den Kreislauf, insbesondere blutdrucksenkende Wirkungen sowie Wirkungen auf die Herzkraft (positiv inotrope Wirksamkeit), aber auch thrombozytenaggregationshemmende Wirkungen.

Die Substanzen vermindern z.B. sowohl den an normotonen narkotisierten als auch an wachen nephrogen hypertonen Hunden gemessenen Blutdruck bei peroraler Gabe dosisabhängig und lang anhaltend (Methodik vgl. B.A. SCHÖLKENS et al., Prostaglandins, Leukotrienes and Medicine, 10, 231–256, 1983).

Die thrombozytenaggregationshemmende Wirkung lässt sich z.B. in vitro im Aggregationstest nach G.V.R. BORN (Nature 194, 927–929, 1962) sowie in vivo durch den Laser-induzierten Thrombosierungstest an Mesenterialgefässen von Ratte und Kaninchen (Methodik in Anlehnung an J. KOVACZ (Mirovascular Research 6, 194, 1973) bzw. J. DUHAULT (Arzneimittelforschung/Drug Research 22/10, 1686–1690, 1972) ) nachweisen.

Die Herzwirkung kann z.B. an narkotisierten oder wachen Hunden, Katzen, Affen oder Minischweinen, aber auch an isolierten Herzpräparationen (z.B. Vorhof, Papillarmuskel oder perfundiertes Ganzherz) von Meerschweinchen oder Katze ermittelt werden, z.B. nach Methoden, wie sie in Arzneimittelforschung, 31 (I) Nr. 1a (1981), Seiten 141 bis 170, beschrieben sind.

Weiterhin sind die Verbindungen der Formel I zytoprotektiv an verschiedenen Organen des menschlichen oder tierischen Körpers wirksam. So hemmen sie z.B. am Magen die Säuresekretion weit stärker als $PGE_2$ (bei intravenöser Gabe) und schützen die Mucosa gegen den Einfluss von ulcerogen wirkenden Substanzen wie Alkohol, Acetylsalicylsäure oder Taurocholsäure. An der Leber schützen sie gegen toxische Einflüsse, z.B. von $CCl_4$ oder Paracetamol, und erhöhen die Überlebenschancen bei fulminantem Leberversagen. Am Herz schützen sie bei Infarkten vor Nektrosen des Herzgewerbes; sie verringern die Infarktgrösse und verkürzen die Heilungszeit. Eine Prinzmetal-Angina kann mit den Substanzen der Formel I behandelt werden. Sie zeigen auch am Gehirn günstige Einflüsse, z.B. wenn Ischämien aufgetreten sind. An der Lunge bewirken sie einen Abfall des Gefässwiderstandes der zentralen Atemwege; daher können sie für die Behandlung von asthmatischen Krankheiten eingesetzt werden.

Ausserdem treten bei den 6-Ketoprostaglandinen der Formel I vasodilatorische, diuretische, bronchienentkrampfende, die Magensaftsekretion, den Lipidabbau und die Noradrenalin-Freisetzung hemmende, sowie Nasenschleimhautabschwellende Eigenschaften auf, die ebenfalls mit hierfür geläufigen Methoden ermittelt werden können. Ferner können die 6-Ketoprostaglandine der allgemeinen Formel I auch die Funktion des corpus luteum, den Eitransport durch den Eileiter, die Nidation und die Fertilität beeinflussen.

Die Verbindungen der allgemeinen Formel I und ihre physiologisch unbedenklichen Salze können daher als Arzneimittelwirkstoffe in der Human- und Veterinärmedizin und auch als Zwischenprodukte zur Herstellung anderer Arzneimittelwirkstoffe verwendet werden.

Gegenstand der Erfindung sind die 6-Ketoprostaglandine der allgemeinen Formel I.

In der allgemeinen Formel I und den anderen Formeln dieser Anmeldung ist eine α-ständige Bindung punktiert und eine β-ständige Bindung keilförmig ausgezogen eingezeichnet. Bindungen, die α- oder β-ständig sein können, sind durch eine Wellenlinie gekennzeichnet. Bevorzugt sind durch eine Wellenlinie gekennzeichnet. Bevorzugt sind Verbindungen, bei denen in der Thioether-Seitenkette die $R^3O$-Gruppe α-ständig und die Gruppe $R^5$ β-ständig ist.

Die Verbindungen der allgemeinen Formel I enthalten drei asymmetrische C-Atome am carbocyclischen Fünfring. In der Thioether-Seitenkette können weitere Asymmetriezentren auftreten.

Die Verbindungen der allgemeinen Formel I können daher in einer Vielzahl stereoisomerer Formen auftreten.

Gegenstand der Erfindung sind neben den einzelnen Racematen und racemischen Gemischen auch die verschiedenen optisch aktiven Isomeren der allgemeinen Formel I.

Gegenstand der Erfindung ist ferner ein Verfahren zur Herstellung der Verbindungen der allgemeinen Formel I sowie ihrer Salze, das dadurch gekennzeichnet ist, dass man eine Verbindung der Formel II

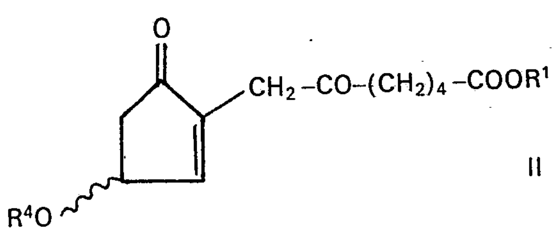

II

worin

$R^1$ und $R^4$ die in Anspruch 1 angegebenen Bedeutungen haben mit einer Verbindung der Formel III

$MS-CH_2-CR^5(OR^3)-D-R^2$ III

worin

M H oder ein Äquivalent eines Kations bedeutet und

D, $R^2$, $R^3$ und $R^5$ die in Anspruch 1 angegebenen Bedeutungen haben umsetzt

und/oder dass man gegebenenfalls eine Verbindung der Formel I, worin $R^1$ und/oder $R^3$ und/oder $R^4$ von H verschieden ist (sind), durch Behandeln mit solvolysierenden Mitteln in eine Verbindung der Formel I, worin $R^1$ und/oder $R^3$ und/oder $R^4$ H ist (sind), überführt und/oder eine Verbindung der Formel I, worin $R^1$=H ist, verestert und/oder eine Verbindung dfer Formel I, worin $R^3$ und/oder $R^4$ H ist (sind), verethert oder verestert und/oder eine als Racemat erhaltene Verbindung der Formel I in ihre Enantiomeren aufspaltet und/oder eine Verbindung der Formel I, worin $R^1$=H ist, durch Umsetzen mit einer Base in eines ihrer Salze überführt.

In den Formeln I und III bedeutet D vorzugsweise eine Bindung sowie Alkylen mit 1, 2 oder 3 C-Atomen, insbesondere Methylen, aber auch Ethylen, Propylen oder 1-Methyl-ethylen. Wenn D cis-Alkenylen mit 2–5 C-Atomen bedeutet, so sind unverzweigte cis-Alkenylen-Gruppen mit 3, 4 oder 5 C-Atomen bevorzugt, insbesondere cis-But-2-enylen. D kann aber auch für eine verzweigte cis-Alkenylen-Gruppe stehen, beispielsweise für 1-Methyl-but-2-enylen. Wenn D Alkinylen mit 2–5 C-Atomen bedeutet, so sind verzweigte Alkinylen-Gruppen mit 3, 4 oder 5 C-Atomen bevorzugt, insbesondere 1-Pentin-1,4-ylen.

Die Gruppe $R^5$ steht insbesondere für Wasserstoff oder Methyl.

$R^1$ bedeutet insbesondere Wasserstoff, ferner einen Alkylrest, vorzugsweise einen unverzweigten Alkylrest mit bis zu 4 C-Atomen, wie Methyl, Ethyl, Propyl oder n-Butyl, aber auch einen verzweigten, wie Isopropyl oder tert.-Butyl. Ferner bedeutet $R^1$ einen Arylrest mit 6–12 C-Atomen wie Phenyl, Tolyl, Biphenylyl oder Naphthyl. Besonders vorteilhaft bedeutet $R^1$ auch den Rest $-C_6H_4-NH-CO-C_6H_5$.

$R^2$ bedeutet z.B. einen Alkylrest mit 1–7 C-Atomen, vorzugsweise einen unverzweigten Alkylrest mit 1–7 C-Atomen, wie Methyl, Ethyl, Propyl, Butyl, Pentyl, Hexyl oder Heptyl. $R^2$ kann aber auch ein verzweigter Alkylrest mit 3–7 C-Atomen sein, wie beispielsweise 1-Methylpentyl, 1,1-Dimethylpentyl, 2-Methylpentyl, 3-Methylpentyl, 3,3-Dimethylpentyl, -Methylpentyl oder 1-Methylhexyl.

Falls $R^2$ einen durch Halogen substituierten Alkylrest mit 1–7 C-Atomen bedeutet, so befindet sich der Halogensubstituent, der vorzugsweise Fluor oder Chlor ist, aber auch Brom oder Jod sein kann, insbesondere in der Endstellung. Als Beispiele seien genannt: Chlormethyl, 2-Chlorethyl, 3-Chlorpropyl, 4-Chlorbutyl, 5-Chlorpentyl, 1-Methyl-4-chlorbutyl, 1-Methyl-5-chlorpentyl, 1,1-Dimethyl-4-chlorbutyl; Fluormethyl, 2-Fluorethyl, 3-Fluorpropyl, 4-Fluorbutyl, 5-Fluorpentyl; 4-Brombutyl, 5-Brombutyl.

$R^2$ bedeutet vorzugsweise auch Cycloalkyl mit 5–6 C-Atomen, wie Cyclopentyl und insbesondere Cyclohexyl. Ferner kann $R^2$ durch Alkyl mit 1–4 C-Atomen, vorzugsweise 1–2 C-Atomen

substituiertes Cycloalkyl mit 5–6 C-Atomen bedeuten, wie beispielsweise 2-Methylcyclohexyl, 3-Methylcyclohexyl, 4-Methylcyclohexyl, 4-Ethylcyclohexyl, 4-Propylcyclohexyl, 4-Isopropylcyclohexyl, 4-Butylcyclohexyl, 4-tert.-Butylcyclohexyl; 2-Methylcyclopentyl, 3-Methylcyclopentyl, 3-Ethylcyclopentyl, 3-Propylcyclopentyl, 3-Butylcyclopentyl.

$R^2$ bedeutet auch vorzugsweise Phenyl oder ein-, zwei- oder dreifach durch F, Cl, Br, Alkyl mit 1–4 C-Atomen, OH, $OCH_3$, oder $CF_3$ substituiertes Phenyl. Wenn $R^2$ ein substituierter Phenylrest ist, so ist er vorzugsweise einfach substituiert, wobei der Substituent in 2-, 3- oder 4-Stellung zu finden ist.

$R^2$ ist daher vorzugsweise auch Phenyl, 3-Fluorphenyl, 4-Fluorphenyl, 2-Chlorphenyl, 3-Chlorphenyl, 4-Chlorphenyl, 3-Bromphenyl, 4-Bromphenyl, 2-Tolyl, 3-Tolyl, 4-Tolyl, 4-Ethylphenyl, 4-Isopropylphenyl, 4-Butylphenyl, 3-Hydroxyphenyl, 4-Hydroxhenyl, 3-Methoxyphenyl, 4-Methoxyphenyl, 2-Trifluormethylphenyl, 3-Trifluormethylphenyl, 4-Trifluormethylphenyl, aber auch beispielsweise 2,4-Dichlor-, 3,4-Dichlor-, 2,4-Dimethyl-, 3,4-Dimethyl-, 2,4-Dimethoxy-, 2,3-Dimethoxy-, 2,4,6-Trimethyl- oder 3,4,5-Trimethoxyphenyl.

$R^2$ bedeutet ferner 2-Pyridyl, 3-Pyridyl, 4-Pyridyl, 1-Naphthyl, 2-Naphthyl, 2-Thienyl oder 3-Thienyl.

Falls D Alkylen mit 1–3 C-Atomen, insbesondere Methylen bedeutet, steht $R^2$ auch vorzugsweise für Alkoxy mit 1–4 C-Atomen, wie Methoxy, Ethoxy, Propyloxy oder Butyloxy; Alkylthio mit 1–4 C-Atomen wie Methylthio, Ethylthio, Propylthio oder Butylthio, Phenoxy oder ein-, zwei- oder dreifach durch F, Cl, Br, Alkyl mit 1–4 C-Atomen, OH, $OCH_3$ oder $CF_3$ substituiertes Phenoxy. Wenn $R^2$ ein substituierter Phenoxyrest ist, so ist er vorzugsweise einfach substituiert, wobei der Substituent in 2-Stellung, insbesondere aber in 3- oder 4-Stellung zu finden ist. Beispiele für solche Reste sind Phenoxy, 3-Fluorphenoxy, 4-Fluorphenoxy, 3-Chlorphenoxy, 4-Chlorphenoxy, 3-Bromphenoxy, 4-Bromphenoxy, 2-Methylphenoxy, 3-Methylphenoxy, 4-Methylphenoxy, 4-Ethylphenoxy, 4-Isopropylphenoxy, 4-Butylphenoxy, 3-Hydroxyphenoxy, 4-Hydroxyphenoxy, 3-Methoxyphenoxy, 4-Methoxyphenoxy, 3-Trifluormethylphenoxy, 4-Trifluormethylphenoxy, aber auch beispielsweise 2,4-Dichlor-, 3,4-Dichlor-, 2,4-Dimethyl-, 3,4-Dimethyl-, 2,4-Dimethoxy-, 2,3-Dimethoxy-, 2,4,6-Trimethyl- oder 3,4,5-Trimethoxyphenoxy.

D–$R^2$ bedeutet demnach vorzugsweise Butyl, Pentyl, Hexyl, Heptyl, Octyl, Nonyl, Decyl; 1-Methylpentyl, 1,1-Dimethylpentyl, 2-Methylpentyl, 3-Methylpentyl, 4-Methylpentyl, 1-Methylhexyl; 4-Chlorbutyl, 4-Chlorpentyl, 5-Chlorpentyl, 1-Methyl-4-chlorbutyl, 1,1-Dimethyl-4-chlorbutyl, 5-Fluorpentyl, 5-Brompentyl; But-2-enyl, Pent-2-enyl, Hex-2-enyl, Pent-3-enyl, Hex-3-enyl, Hex-4-enyl, Hept-4-enyl; 1-Methylpent-3-inyl; Cyclopentyl, Cyclohexyl, 3-Methylcyclohexyl, 4-Methylcyclohexyl, 3-Ethylcyclohexyl, 4-Isopropylcyclohexyl, Cyclohexylmethyl, 2-Cyclohexylethyl, Cyclopentylmethyl; Phenyl, 2-Fluorphenyl, 3-Fluorphenyl, 4-Fluorphenyl, 2-Chlorphenyl, 3-Chlorphenyl, 4-Chlorphenyl, 2-Tolyl, 3-Tolyl, 4-Tolyl, 3-Methoxyphenyl, 4-Methoxyphenyl, 2-Trifluormethylphenyl, 3-Trifluormethylphenyl, 4-Trifluormethylphenyl, Benzyl, 4-Fluorbenzyl, 3-Chlorbenzyl, 4-Chlorbenzyl, 4-Methylbenzyl, 4-Methoxybenzyl, 3-Trifluormethylbenzyl, 4-Trifluormethylbenzyl; 2-Phenylethyl, 2-(3-Chlorphenyl)-ethyl, 2-(3-Trifluormethylphenyl)-ethyl; 4-Pyridyl, 4-Pyridylmethyl, 2-Naphthyl, 2-Naphthylmethyl, 2-Thienyl, 2-Thienylmethyl, 3-Thienyl, 3-Thienylmethyl-, Ethoxymethyl, Propyloxymethyl, Butyloxymethyl, 2-Methoxyethyl, 2-Ethoxyethyl, 2-Propyloxyethyl, 3-Methoxypropyl, 3-Ethoxypropyl; Ethylthiomethyl, Propylthiomethyl, Butylthiomethyl, 2-Methylthioethyl, 2-Ethylthioethyl, 2-Propylthioethyl, 3-Methylthiopropyl, 3-Ethylthiopropyl; Phenoxymethyl, 3-Fluorphenoxymethyl, 4-Fluorphenoxymethyl, 3-Chlorphenoxymethyl, 4-Chlorphenoxymethyl, 4-Methoxyphenoxymethyl, 3-Trifluormethylphenoxymethyl, 4-Trifluormethylphenoxymethyl.

Die Reste $R^3$ und $R^4$ bedeuten vorzugsweise H. Weiterhin können diese beiden Reste, insbesondere aber der Rest $R^3$ für Hydroxyschutzgruppen stehen, insbesondere für Alkyl mit 1–6 C-Atomen, vorzugsweise tert.-Butyl, Methyl, Ethyl oder Propyl, ferner Isopropyl, Butyl, Isobutyl, sek.-Butyl, Pentyl, Hexyl oder Heptyl; für Tetrahydropyran-2-yl; für Trialkylsilyl mit insgesamt 3–12 C-Atomen, vorzugsweise Trimethylsilyl, tert.-Butyldimethyl-silyl, Triethylsilyl, Triisopropylsilyl, Di-tert.-butyl-methylsilyl; Aryldialkylsilyl mit insgesamt 8–18 C-Atomen, vorzugsweise Phenyldimethylsilyl; Alkoxymethyl mit 2–5 C-Atomen wie Methoxymethyl, Ethoxymethyl, tert.-Butoxymethyl; Aryloxymethyl mit 7–11 C-Atomen wie Phenoxymethyl; Acyl mit 1–10 C-Atomen, insbesondere Alkanoyl mit 1–6 C-Atomen wie Formyl, Acetyl, Trimethylacetyl, tert.-Butylacetyl, oder Aroyl mit 7–10 C-Atomen wie Benzoyl, ferner Kohlensäureestergruppen wie tert.-Butyloxycarbonyl oder Benzyloxycarbonyl.

Als Salze der Verbindungen der Formel I kommen vorzugsweise physiologisch unbedenkliche Metall- und Ammoniumsalze, insbesondere die Natrium-, Kalium-, Magnesium-, Calcium- und Ammoniumsalze in Betracht, ferner substituierte Ammoniumsalze, wie z.B. die Monoethanol- und Triethanolammonium-, Cyclohexylammonium und Dibenzylethylendiammonium-Salze.

Dementsprechend sind Gegenstand der Erfindung insbesondere diejenigen Verbindungen der allgemeinen Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ij ausgedrückt werden, die der allgemeinen Formel I entsprechen, worin jedoch

in Ia D eine Bindung oder $-CH_2-$ bedeutet;
in Ib $R^1$ H, Methyl, Ethyl oder $-C_6H_4-NH-CO-C_6H_5$ bedeutet;
in Ic $R^2$ Alkyl mit 1–6 C-Atomen, Cyclohexyl, Phenyl, durch F, Cl, $CH_3$, $OCH_3$ oder $CF_3$ einfach substituiertes Phenyl oder Naphthyl bedeutet;
in Id $R^3$ H und
$R^4$ H, tert.-Butyl, tert.-Butoxymethyl, Tetrahydropyran-2-yl, Trialkylsilyl mit insgesamt 3–6 C-Atomen oder Alkanoyl mit 2–6 C-Atomen bedeutet;
in Ie D eine Bindung, $-CH_2-$ oder 1-Pentin-1,4-ylen,
$R^1$ H oder Benzamidophenyl,
$R^2$ Alkyl mit 1–7 C-Atomen, Phenyl, Chlorphenyl oder, falls D $-CH_2-$ bedeutet, auch Chlorphenoxy,
$R^3$ H
$R^4$ H, tert.-Butyl, tert.-Butoxymethyl, Tetrahydropyran-2-yl, Trialkylsilyl mit insgesamt 3–6 C-Atomen oder Alkanoyl mit 2–6 C-Atomen und
$R^5$ H oder $CH_3$ bedeuten;
in If D eine Bindung, $-CH_2-$ oder 1-Pentin-1,4-ylen,
$R^1$ H oder p-Benzamidophenyl,
$R^2$ Methyl, Pentyl, Hexyl, 1-Methylpentyl, 1-Methylhexyl, Phenyl, o-Chlorphenyl, oder, falls D $-CH_2-$ bedeutet, auch m-Chlorphenoxy,
$R^3$ H
$R^4$ H oder tert.-Butyldimethylsilyl und
$R^5$ H oder $CH_3$ bedeuten;
in Ig D eine Bindung,
$R^1$ H oder p-Benzamidophenyl,
$R^2$ Pentyl, Hexyl, 1-Methylpentyl oder 1-Methylhexyl,
$R^3$ H
$R^4$ H oder tert.-Butyldimethylsilyl und
$R^5$ H oder $CH_3$ bedeuten;
in Ih D eine Bindung oder $-CH_2-$,
$R^1$ H oder p-Benzamidophenyl,
$R^2$ Phenyl, o-Chlorphenyl oder, falls D $-CH_2-$ bedeutet, auch m-Chlorphenoxy,
$R^3$ H
$R^4$ H oder tert.-Butyldimethylsilyl und
$R^5$ H oder $CH_3$ bedeuten;
in Ii D eine Bindung,
$R^1$ H oder p-Benzamidophenyl, 1-Me-
$R^2$ thylhexyl,
$R^3$ H
$R^4$ H, tert.-Butoxymethyl, Tetrahydropyran-2-yl, Trialkylsilyl mit insgesamt 3–6 C-Atomen oder Alkanoyl mit 2–6 C-Atomen und
$R^5$ H bedeuten;
in Ij D eine Bindung,
$R^1$ H oder p-Benzamidophenyl,
$R^2$ 1-Methylhexyl,
$R^3$ H
$R^4$ H oder tert.-Butyldimethylsilyl und
$R^5$ H bedeuten.

Die Herstellung der Verbindungen der allgemeinen Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe, insbesondere die der allgemeinen Formeln II und III können gewünschtenfalls auch in situ gebildet werden, derart, dass man sie aus dem Reaktionsgemisch nicht isoliert, sondern sofort weiter zu den Verbindungen der allgemeinen Formel I umsetzt.

Die Umsetzung einer Verbindung der Formel II mit einem Thiol oder Thiolat der Formel III erfolgt in der Regel in Gegenwart eines basischen Katalysators und in Anwesenheit oder Abwesenheit eines inerten Lösungsmittels bei Temperaturen zwischen etwa $-50$ und $+20°$, vorzugsweise zwischen $-30$ und $0°$.

Als Lösungsmittel eignen sich vorzugsweise Alkohole wie Methanol, Ethanol, Propanol, Isopropanol, Butanol, tert.-Butylalkohol, Amylalkohol, 2-Methoxyethanol oder 2-Ethoxyethanol; Ether wie Diethylether, Tetrahydrofuran (THF), Dioxan oder Ethylenglykoldimethylether, chlorierte Kohlenwasserstoffe wie Methylenchlorid oder Chloroform; oder auch Wasser.

Geeigente basische Katalysatoren sind z.B. Alkalimetall- oder Erdalkalimetallhydroxide wie NaOH, KOH oder $Ca(OH)_2$; Alkalimetallalkoholate, wie $NaOCH_3$, $NaOC_2H_5$ oder KO-tert.-$C_4H_9$; basische Salze, vorzugsweise Carbonate oder Acetate wie $K_2CO_3$ oder $NaOCOCH_3$; Ammoniak, Amine, vorzugsweise sekundäre oder tertiäre Amine wie Triethylamin, Diisopropylamin, Dicyclohexylamin, Dimethylanilin, Piperidin, 2,6-Dimethylpiperidin, 2,2,6,6-Tetramethylpiperidin, Pyrrolidin, Pyridin, Chinolin, Diazabicyclo-[2.2.2]-octan oder Diazabicyclo[3.4.0]-nonen; aber auch primäre Amine wie tert.-Butylamin oder Cyclohexylamin; oder quaternäre Ammoniumhydroxide wie Tetramethylammoniumhydroxid oder insbesondere Benzyltrimethylammoniumhydroxid. Man kann auch eines der genannten Amine, insbesondere ein sekundäres oder tertiäres Amin, gleichzeitig als Lösungsmittel verwenden und so in Abwesenheit der genannten inerten Lösungsmittel arbeiten. Besonders zweckmässig arbeitet man unter einer Inertgasatmosphäre, beispielsweise unter Stickstoff.

Verbindungen der Formel I bzw. Verbindungen, die der Formel I entsprechen, worin $R^1$ und/oder $R^3$ und/oder $R^4$ von H verschieden ist (sind), können durch Behandeln mit solvolysierenden Mitteln, vorzugsweise unter neutralen oder sauren Bedingungen, in Verbindungen der Formel I, worin $R^1$ und/oder $R^3$ und/oder $R^4$ H ist (sind), übergeführt werden.

Als solvolysierende Mittel verwendet man vorzugsweise hydrolysierende Mittel wie Wasser oder Wasser im Gemisch mit organischen Lösungsmitteln, in Abwesenheit oder vorzugsweise in Anwesenheit eines sauren Katalysators. Als organische Lösungsmittel kommen z.B. in Frage Alkohole wie Methanol, Ethanol, Propanol, Isopropanol, Butanol, tert.-Butylalkohol, Amylalkohol, 2-Methoxyethanol oder 2-Ethoxyethanol; Ether wie Diethylether, THF, Dioxan oder Ethylenglykoldimethylether, Säuren wie Ameisensäure, Essigsäure, Propionsäure oder Buttersäure; Ester wie Ethylacetat oder Butylacetat; Ketone wie Aceton; Amide wie Dimethylformamid (DMF) oder Phosphorsäurehexamethyltriamid; Nitrile wie Acetonitril; Sulfoxide wie Dimethyl Sulfoxid (DMSO); Sulfone wie Tetrahydrothiophen-S,S-dioxid; sowie Gemische dieser Lösungsmittel untereinander und/oder mit Wasser.

Als saure Katalysatoren eignen sich bei einer Solvolyse z.B. anorganische Säuren, beispielsweise Salzsäure, Schwefelsäure, Phosphorsäure, Fluorwasserstoffsäure, Bromwasserstoffsäure, oder starke organische Säuren wie Chloressigsäure, Trichlor- oder Trifluoressigsäure, Methan-, Ethan-, Benzol- oder p-Toluolsulfonsäure. Als basische Katalysatoren verwendet man zweckmässig Alkalimetall- oder Erdalkalimetallhydroxide wie Natrium-, Kalium- oder Calciumhydroxid, oder basische Salze, wie Natrium- oder Kaliumcarbonat. Auch organische Basen, beispielsweise Ethyl-, Diethyl-, Triethyl-, Isopropyl-, n-Butyl- oder Tri-n-butylamin, Dimethylanilin, Pyrrolidin, Piperidin, Morpholin, Pyridin, α-Picolin oder Chinolin, oder quartäre Ammoniumhydroxide, wie z.B. Tetramethylammoniumhydroxid oder Benzyltrimethylammoniumhydroxid können als basische Katalysatoren verwendet werden. Ein Überschuss des Katalysators kann auch an Stelle eines Lösungsmittels verwendet werden. Die Solvolyse wird zweckmässig bei Temperaturen zwischen etwa –50 und 20° vorgenommen, vorzugsweise zwischen –15 und 0°.

Insbesondere können auch Ester der Formel I ($R^1$=H) durch Hydrolyse in Säuren der Formel I ($R^1$=H) umgewandelt werden, z.B. durch basische Hydrolyse wie oben angegeben, bevorzugt unter milden Bedingungen. Auch eine enzymatische Spaltung der Ester ist möglich, z.B. mit einer Lipase in wässriger Suspension bei etwa 0–30°.

Weiterhin können insbesondere Silylether der Formel I ($R^3$ und/oder $R^4$ = Trialkylsilyl oder Aryldialkylsilyl) durch Hydrolyse in die entsprechenden freien Alkohole der Formel I ($R^3$ und/oder $R^4$ =H) umgewandelt werden, vorzugsweise mit HF in wässrigem Acetonitril bei Temperaturen zwischen etwa 0 und 30°.

Eine Säure der Formel I ($R^1$=H) kann durch Umsetzen mit einem entsprechenden veresternden Mittel in einen Ester der Formel I ($R^1$ = Alkyl mit 1–4 C-Atomen, Aryl mit 6–12 C-Atomen oder $-C_6H_4-NH-CO-C_6H_5$) übergeführt werden. Veresternde Mittel sind beispielsweise Alkohole mit bis zu 4 C-Atomen, vorzugsweise in Gegenwart einer anorganischen oder organischen Säure, wie HCl, HBr, HJ, $H_2SO_4$, $H_3PO_4$, Trifluoressigsäure, einer Sulfonsäure wie Benzolsulfonsäure oder p-Toluolsulfonsäure, oder eines sauren Ionenaustauschers; Diazoalkane mit bis zu 4 C-Atomen, vorzugsweise Diazomethan; Olefine (z.B. Isobutylen), vorzugsweise in Gegenwart von sauren Katalysatoren (z.B. $ZnCl_2$, $BF_3$, $H_2SO_4$, Arylsulfonsäure, Pyrophorphorsäure, Borsäure, Oxalsäure); Alkylhalogenide mit bis zu 4 C-Atomen, vorzugsweise Bromide, wie Ethyl-, Propyl-, Isopropyl- oder Butylbromid, aber auch die entsprechenden -chloride oder -jodide; Carbonsäure- oder Sulfonsäurealkylester, wobei der Säurerest beliebig sein kann und der Alkylrest bis zu 4 C-Atome enthält, vorzugsweise Methyl-, Ethyl-, Propyl-, Isopropyl- oder Butylacetat, -formiat, -methylsulfonat, -ethylsulfonat oder -p-toluolsulfonat, und insbesondere auch Dialkylschwefelsäureester mit bis zu 4 C-Atomen, wie Dimethylsulfat oder Diethylsulfat.

Die Veresterung erfolgt zweckmässig in einem inerten, vorzugsweise wasserfreien Lösungsmittel, beispielsweise einem Ether wie Diethylether oder THF, einem Alkohol, vorzugsweise einem der genannten Alkohole mit bis zu 4 C-Atomen, oder auch in einem Kohlenwasserstoff, wie Petrolether, Hexan, Benzol oder Toluol, oder in Gemischen dieser Lösungsmittel bei Temperaturen zwischen –10° und 40°, vorzugsweise bei Raumtemperatur. Die Reaktionszeiten liegen in der Regel zwischen 30 Minuten und 20 Stunden.

Aromatische Ester der Formel I ($R^1$ = Aryl mit 6–12 C-Atomen oder $-C_6H_4-NH-CO-C_6H_5$) können aus Säuren der Formel I ($R^1$ = H) durch Umsetzen mit einem Phenol (wie beispielsweise Phenol, m-Kresol, p-Kresol, p-Ethylphenol, α-Naphthol, β-Naphthol, p-Phenylphenol) oder einer Verbindung der Formel $HO-C_6H_4-NH-CO-C_6H_5$ hergestellt werden. Die letztgenannten Verbindungen sind bekannt, beispielsweise aus der DE-A-26 44 972.

Die Veresterung einer Säure der Formel I ($R^1$ = H) mit einem Phenol kann nach an sich bekannten Methoden durchgeführt werden; vorzugsweise arbeitet man in Gegenwart eines wasserbindenden Mittels, z.B. eines Carbodiimids, wie Dicyclohexylcarbodiimid, und in einem inerten organischen Lösungsmittel, vorzugsweise einem Ether wie Diethylether, 1,2-Dimethoxyethan, THF oder Dioxan; oder einem Halogenkohlenwasserstoff wie Methylenchlorid oder 1,2-Dichlorethan; oder in Gemischen dieser Lösungsmittel mit DMF. Die Reaktionstemperaturen liegen beispielsweise etwa zwischen –20 und 100°.

In analoger Weise können Verbindungen der Formel I, worin $R^3$ und/oder $R^4$ ist (sind), mit einer Säure, die 1–10 C-Atome enthält, oder mit einem reaktionsfähigen Derivat einer solchen Säure verestert werden. Als reaktionsfähige Säurederivate eignen sich z.B. die Anhydride und die Halogenide, z.B. Chloride oder Bromide der Säuren.

So gelingt z.B. eine Acylierung der Hydroxyverbindungen der Formel I durch Behandeln mit überschüssigem Anhydrid, z.B. Acetanhydrid in Gegenwart einer Base wie Triethylamin, Imidazol oder Pyridin bei Temperaturen zwischen etwa

−20 und 50°, vorzugsweise zwischen 0 und 30°. Dabei werden sekundäre OH-Gruppen bevorzugt vor tertiären verestert.

In ähnlicher Weise gelingt eine Verätherung der Hydroxyverbindungen der Formel I ($R^3$ und/oder $R^4$ = H) mit entsprechenden Alkyl-, Trialkylsilyl-, Aryldialkylsilyl-, Alkoxymethyl- oder Aryloxymethyl-halogeniden, vorzugsweise -chloriden, vorteilhaft in Gegenwart eines inerten Lösungsmittels wie DMF.

Die Verbindungen der Formel I werden meist als Gemische verschiedener stereoisomerer Formen erhalten, d.h. in der Regel als Gemische von Racematen. Racemate können aus den Racematgemischen isoliert und rein erhalten werden, beispielsweise durch Umkristallisieren der Verbindungen selbst oder von gut kristallisierenden Derivaten, insbesondere aber mit Hilfe chromatographischer Methoden, wobei sowohl adsorptionschromatographische oder verteilungschromatographische Methoden als auch Mischformen in Frage kommen.

Die Racemate können nach bekannten Methoden, wie sie in der Literatur angegeben sind, in ihre optischen Antipoden getrennt werden. Die Methode der chemischen Trennung wird bevorzugt.

So kann man eine optisch aktive Base mit der Carboxylgruppe einer Verbindung der Formel I ($R^1$ = H) umsetzen. Zum Beispiel kann man die diastereomeren Salze mit optisch aktiven Aminen, wie Chinin, Cinchonidin, Brucin, Cinchonin, Hydroxyhydrindamin, Morphin, 1-Phenylethylamin, 1-Naphthylethylamin, Phenyloxynaphthylmethylamin, Chinidin, Strychnin, basischen Aminosäuren, wie Lysin, Arginin oder Aminosäureestern bilden. In ähnlicher Weise lassen sich Ester-Diastereomere durch Veresterung von Carbonsäuren der Formel ($R^1$=H) mit optisch aktiven Alkoholen, wie Borneol, Menthol, Octanol-2, herstellen. Die anfallenden diastereomeren Salze bzw. Ester werden durch Kristallisation getrennt und die optisch aktiven Verbindungen aus dem Gemisch in Freiheit gesetzt.

Aber auch die anderen in den Verbindungen der Formel I vorhandenen funktionellen Gruppen können zur Bildung von Diastereomeren herangezogen werden. So kann man z.B. OH-Gruppen mit optisch aktiven Säuren wie (+)- und (−)-Weinsäure oder Camphersäure verestern und aus diesen Derivaten die reinen Enantiomeren gewinnen.

Weiterhin ist es natürlich möglich, optisch aktive Verbindungen nach den beschriebenen Methoden zu erhalten, indem man Ausgangsstoffe verwendet, die bereits optisch aktiv sind.

Ferner kann man die freien Säuren der Formel I ($R^1$=H) durch Umsetzung mit einer Base in eines ihrer Salze, insbesondere eines ihrer physiologisch unbedenklichen Metall- oder Ammoniumsalze überführen.

Die Ausgangsverbindungen zur Herstellung der erfindungsgemässen Verbindungen der Formel I nach den vorstehend beschriebenen Methoden sind teils bekannt, zum grössten Teil jedoch neu. Neue Ausgangsverbindungen können aus bekannten Verbindungen in Analogie zu bekannten Verfahren hergestellt werden, neue Verbindungen der Formel II beispielsweise wie folgt:

1α-Hydroxy- 2α-(6-carboxy-hex-2-enyl) 3α,-4α-oxidocyclopentan, das aus Synth. Comm. 4 (6), 317 (1974) bekannt, ist, oder dessen Ester werden mit N-Bromsuccinimid in 2-Oxa-3-(1-brom- 4-carboxybutyl)- 6,7-oxido- bicyclo (3,3,0) -octan oder dessen Ester übergeführt. Aus diesen sind mit starken Basen, z.B. K-tert.butylat, 6-Oxo-7- (2α,3α-oxido-5α-hydroxy-1α-cyclopentyl)- heptansäure oder deren Ester herstellbar. Oxydation, z.B. mit $CrO_3$, führt zu den entsprechenden 2α,3α-Oxido-5-oxo-1α-cyclopentylderivaten, die durch Spaltung des Epoxids und Dehydratisierung Verbindungen der Formel II liefern, in denen $R^4$ H bedeutet. Durch übliche Veretherung oder Veresterung sind daraus die übrigen Verbindungen der Formel II zugänglich. Bei den Verbindungen der Formel III handelt es sich um 2-Hydroxythiole oder deren Alkalimetall-, Erdalkalimetall- oder gegebenenfalls substituierte Ammoniumsalze. Die meisten Thiole der Formel III sind bekannt, beispielsweise aus der DE-A-22 56 537, der DE-A-24 22 924 und der DE-A-26 44 972. Neue Verbindungen der Formel III können aus bekannten Verbindungen in Analogie zu bekannten Verfahren hergestellt werden, beispielsweise aus den entsprechenden Oxiranen durch Umsetzen mit $H_2S$ und gegebenenfalls anschliessende Überführung in ihre Alkalimetall-, Erdalkalimetall- oder gegebenenfalls substituierte Ammoniumsalze. Ebenso können die Oxirane direkt mit Alkalimetall-, Erdalkalimetall- oder Ammoniumhydrogensulfiden umgesetzt werden, wobei dann direkt die Verbindungen der Formel III mit M ungleich H erhalten werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale) oder parenterale Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs- Stabilisierungs- und/ oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten verwendet werden, insbesondere von allen Formen der Hypertonie sowie von Herzinsuffizienz, ferner zur Beeinflussung der Thrombozytenfunktion im Sinne einer Aggregations-(Adhäsions)-Hemmung oder Erkrankungen des atherosklerotischen Formenkreises.

Gegenstand der Erfindung ist auch die Verwendung der Verbindungen der Formel I bei der therapeutischen Behandlung des menschlichen oder tierischen Körpers. Dabei werden die erfindungsgemässen Substanzen in der Regel in Analogie zu bekannten im Handel befindlichen Präparaten ähnlicher Indikation verabreicht, vorzugsweise in Dosierungen zwischen etwa 0,1 und 100 mg, insbesondere zwischen 0,5 und 50 mg pro Dosierungseinheit. Die tägliche Dosierung liegt vorzugsweise zwischen etwa 0,01 und 1 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite, das Fehlen unerwünschter Nebenwirkungen und periphere Entlastung aus.

Vor- und nachstehend sind alle Temperaturen in °C angegeben. Rf-Werte an Kieselgel mit Ethylacetat als Elutionsmittel, sofern nicht anders angegeben.

«Übliche Aufarbeitung» bedeutet: man dampft gegebenenfalls unter vermindertem Druck ein, gibt Wasser bzw. wässerige Säure und $CH_2Cl_2$ zu, trennt ab, trocknet die organische Phase mit $MgSO_4$, dampft ein und reinigt durch Chromatographie an Kieselgel.

Beispiel 1

Eine Lösung von 32,4 g 2-Hydroxy-2-methyl-heptanthiol und 112 ml Diisopropylamin in 320 ml THF wird 2 Std. auf 40° erwärmt, dann auf −25° abgekühlt und mit einer vorgekühlten Lösung von 54,9 g 6-Oxo-7-(3-tert.-butyldimethyl-silyloxy-5-oxo-1-cyclopentenyl)- heptansäure-p-benzamidophenylester [F. 136–137°; erhältlich durch Reaktion von 2α-(6-Carboxy-2-cis-hexenyl)- 3α,4α-oxidocyclopentan-1α-ol mit N-Bromsuccinimid in wässerigem Aceton zu 1-Oxa-2- (1-brom-4-carboxybutyl)- 5,6-oxidobicyclo (3,3,0)octan, Umsetzung mit K-tert.-Butylat in THF zu 6-Oxo-7- (2α,3α-oxido-5α-hydroxy- 1α-cyclopentyl)-heptansäure (p-Benzamidophenylester, F. 134–135°), Oxidation mit $CrO_3$ in wässrigem Aceton zu 6-Oxo-7- (2α,3α-oxido-5-oxo-1α-cyclopentyl)- heptansäure (F. 93–94°; p-Benzamidophenylester, F. 140–141°), Reaktion mit p-Benzamidophenol/Dicyclohexylcarbodiimid in Gegenwart von p-Toluolsulfonsäure zu 6-Oxo-7- (3-hydroxy-5-oxo-1-cyclopentenyl)- heptansäure- p-benzamidophenylester (F. 143–146°) und Veretherung mit tert.-Butyl-dimethyl-chlorsilan] in 450 ml THF langsam versetzt. Man rührt 24 Std. bei −25°, giesst dann in eiskalte Citronensäurelösung, arbeitet wie üblich auf und erhält 6,9-Dioxo-11α-tert.-Butyldimethyl- silyloxy-13-thia- 15-hydroxy- 15-methyl-prostansäure- p-benzamidophenylester, Isomerengemisch, F. 112°. Dieses Gemisch kann chromatographisch in die 15α- und 15β-Isomeren getrennt werden.

Analog sind durch Anlagerung entsprechender Thiole der Formel III an entsprechende Cyclopentenone die folgenden Prostansäure-p-benzamido-phenylester erhältlich:

6,9-Dioxo-11α-tert.-butyldimethyl-silyloxy- 13-thia-15-hydroxy-, Isomerengemisch, F. 141–142°-
6,9-Dioxo-11α-tert.-butyldimethyl-silyloxy- 13-thia-15β-hydroxy-, F. 137–138°
6,9-Dioxo-11α-tert.-butyldimethyl-silyloxy- 13-thia-15α-hydroxy-, F. 146–147°
6,9-Dioxo-11α-tert.-butyldimethyl-silyloxy- 13-thia-15-hydroxy-20-methyl-, Isomerengemisch, F. 142–143°
6,9-Dioxo-11α-tert.-butyldimethyl-silyloxy- 13-thia-15α-hydroxy-20-methyl-, F. 135–136°
6,9-Dioxo-11α-tert.-butyldimethyl-silyloxy-13-thia-15β-hydroxy-20-methyl-, F. 146°
6,9-Dioxo-11α-tert.-butyldimethyl-silyloxy- 13-thia-15-hydroxy-16-methyl-, Isomerengemisch, F. 125–126°
6,9-Dioxo-11α-tert.-butyldimethyl-silyloxy- 13-thia-15α-hydroxy-16-methyl-, F. 125–126°
6,9-Dioxo-11α-tert.-butyldimethyl-silyloxy-13-thia-15β-hydroxy-16-methyl-, F. 132–133°
6,9-Dioxo-11α-tert.-butyldimethyl-silyloxy- 13-thia-15-hydroxy-16,20-dimethyl-, Isomerengemisch, F. 124–125°
6,9-Dioxo-11α-tert.-butyldimethyl-silyloxy- 13-thia-15α-hydroxy-16,20-dimethyl-, F. 126–127°
6,9-Dioxo-11α-tert.-butyldimethyl-silyloxy- 13-thia-15β-hydroxy-16,20-dimethyl-, F. 131–132°
6,9-Dioxo-11α-tert.-butyldimethyl-silyloxy- 13-thia-15-hydroxy-20-ethyl-, Isomerengemisch, F. 143°
6,9-Dioxo-11α-tert.-butyldimethyl-silyloxy- 13-thia-15α-hydroxy-20-ethyl-, F. 138°
6,9-Dioxo-11α-tert.-butyldimethyl-silyloxy-13-thia-15β-hydroxy-20-ethyl-, F. 147°
6,9-Dioxo-11α-tert.-butyldimethyl-silyloxy-13-thia-15-hydroxy-15,20-dimethyl-, Isomerengemisch, F. 114°
6,9-Dioxo-11α-tert.-butyldimethyl-silyloxy- 13-thia-15α-hydroxy-15,20-dimethyl
6,9-Dioxo-11α-tert.-butyldimethyl-silyloxy- 13-thia-15β-hydroxy-15,20-dimethyl
6,9-Dioxo-11α-tert.-butyldimethyl-silyloxy-13-thia-15-hydroxy-16,16,20-trimethyl-, Isomerengemisch
6,9-Dioxo-11α-tert.-butyldimethyl-silyloxy- 13-thia-15α-hydroxy-16,16,20-trimethyl-6,9-Dioxo-11α-tert.-butyldimethyl-silyloxy- 13-thia-15β-hydroxy-16,16,20- trimethyl-6,9-Dioxo-11α-tert.-butyldimethyl-silyloxy- 13-thia-15-hydroxy-15-methyl-20-ethyl-, Isomerengemisch, F. 115°
6,9-Dioxo-11α-tert.-butyldimethyl- silyloxy-13-thia-15α-hydroxy-15- methyl-20-ethyl-
6,9-Dioxo-11α-tert.-butyldimethyl-silyloxy-13-thia-15β-hydroxy-15-methyl-29-ethyl-6,9-Dioxo-11α-tert.-butyldimethyl-silyloxy- 13-thia-15-hydroxy-16-methyl- 20-ethyl-, Isomerengemisch, F. 128°
6,9-Dioxo-11α-tert.-butyldimethyl-silyloxy-13-thia-15α-hydroxy-16-methyl-20-ethyl-, F. 127°
6,9-Dioxo-11α-tert.-butyldimethyl-silyloxy-13-thia-15β-hydroxy-16-methyl-20-ethyl-, F. 133°
6,9-Dioxo-11α-tert.-butyldimethyl-silyloxy-13-thia-15-hydroxy-16,17,18,19,20-pentanor- 15-cyclohexyl, Isomerengemisch, F. 158°
6,9-Dioxo-11α-tert.-butyldimethyl-silyloxy-13-thia-15α-hydroxy-16,17,18,19,20-pentanor -15-cyclohexyl-, F. 150°
6,9-Dioxo-11α-tert.-butyldimethyl-silyloxy- 13-thia-15β-hydroxy-16,17,18,19,20-pentanor-15-cyclohexyl-, F. 167°
6,9-Dioxo-11α-tert.-butyldimethyl-silyloxy- 13-thia-15-hydroxy 18,19,20-trinor-17-cyclohexyl-, Isomerengemisch, F. 156°
6,9-Dioxo-11α-tert.-butyldimethyl-silyloxy- 13-thia-15α-hydroxy- 18,19,20- trinor-17-cyclohexyl-, F. 149°
6,9-Dioxo-11α-tert.-butyldimethyl-silyloxy- 13-thia-15β-hydroxy- 18,19,20-trinor-17-cyclohexyl-, F. 160°
6,9-Dioxo-11α-tert.-butyldimethyl-silyloxy- 13-thia-15-hydroxy- 16,17,18,19,20- pentanor-15-phenyl-, Isomerengemisch, F. 146°
6,9-Dioxo-11α-tert.-butyldimethyl-silyloxy- 13-thia-15α-hydroxy- 16,17,18,19,20-pentanor-15-phenyl-, F. 150°
6,9-Dioxo-11α-tert.-butyldimethyl-silyloxy- 13-thia-15β-hydroxy- 16,17,18,19,20-pentanor-15-phenyl-, F. 137°
6,9-Dioxo-11α-tert.-butyldimethyl-silyloxy- 13-thia-15-hydroxy- 18,19,20-trinor-16-phenyl-, Isomerengemisch, F. 125°
6,9-Dioxo-11α-tert.-butyldimethyl-silyloxy- 13-thia-15α-hydroxy- 18,19,20-trinor-16-phenyl-, F. 105°
6,9-Dioxo-11α-tert.-butyldimethyl-silyloxy- 13-thia-15β-hydroxy- 18,19,20-trinor-16-phenyl-, F. 143°
6,9-Dioxo-11α-tert.-butyldimethyl-silyloxy- 13-thia-15-hydroxy-16-methyl-18,19,20-trinor-17-phenyl-, Isomerengemisch
6,9-Dioxo- 11α-tert.-butyldimethyl-silyloxy- 13-thia-15α-hydroxy16-methyl-18,19,20- trinor-17-phenyl-
6,9-Dioxo-11α-tert.-butyldimethyl-silyloxy- 13-thia-15β-hydroxy- 16-methyl-18,19,20-trinor-17-phenyl-
6,9-Dioxo-11α-tert.-butyldimethyl-silyloxy- 13-thia-15-hydroxy-16,16- dimethyl-18,19,20-trinor- 17-phenyl-, Isomerengemisch,
6,9-Dioxo-11α-tert.-butyldimethyl-silyloxy- 13-thia-15α-hydroxy-16,16- dimethyl-18,19,20-trinor-17-phenyl-
6,9-Dioxo-11α-tert.-butyldimethyl-silyloxy- 13-thia-15β-hydroxy- 16,16-dimethyl-18,19,20- trinor-17-phenyl-
6,9-Dioxo-11α-tert.-butyldimethyl-silyloxy-13-thia-15-hydroxy-16,17,18,19,20- pentanor-15-p-fluorphenyl, Isomerengemisch
6,9-Dioxo-11α-tert.-butyldimethyl-silyloxy-13-thia-15α-hydroxy- 16,17,18,19,20- pentanor-15- p-fluorphenyl-
6,9-Dioxo-11α-tert.-butyldimethyl-silyloxy- 13-thia-15βhydroxy- 16,17,18,19,20- pentanor-15-p-fluorphenyl-
6,9-Dioxo-11α-tert.-butyldimethyl-silyloxy- 13-thia-15-hydroxy-16,17,18,19,20- pentanor-15-o-chlorphenyl-, Isomerengemisch, F. 148°
6,9-Dioxo-11α-tert.-butyldimethyl-silyloxy- 13-thia-15α-hydroxy-16,17,18,19,20- pentanor-15-o-chlorphenyl-, F. 165°
6,9-Dioxo-11α-tert.-butyldimethyl-silyloxy- 13-thia-15β-hydroxy-16,17,18,19,20- pentanor-15-o-chlorphenyl-, F. 137°
6,9-Dioxo-11α-tert.-butyldimethyl-silyloxy- 13-thia-15-hydroxy-16,17,18,19,20- pentanor-15-m-chlorphenyl-, Isomerengemisch, F. 145°

6,9-Dioxo-11α-tert.-butyldimethyl-silyloxy- 13-thia-15α-hydroxy-16,17,18,19,20- pentanor-15-m-chlorphenyl-, F. 150°
6,9-Dioxo-11α-tert.-butyldimethyl-silyloxy- 13-thia-15β-hydroxy-16,17,18,19,20- pentanor-15-m-chlorphenyl-, F. 133°
6,9-Dioxo-11α-tert.-butyldimethyl-silyloxy- 13-thia-15-hydroxy-16,17,18,19,20- pentanor-15-p-chlorphenyl-, Isomerengemisch, F. 139°
6,9-Dioxo-11α-tert.-butyldimethyl-silyloxy- 13-thia-15α-hydroxy-16,17,18,19,20- pentanor-15-p-chlorphenyl-, F. 143°
6,9-Dioxo-11α-tert.-butyldimethyl-silyloxy- 13-thia-15β-hydroxy-16,17,18,19,20- pentanor-15-p-chlorphenyl-, F. 133°
6,9-Dioxo-11α-tert.-butyldimethyl-silyloxy- 13-thia-15-hydroxy-16,17,18,19,20- pentanor-15-p-bromphenyl-, Isomerengemisch
6,9-Dioxo-11α-tert.-butyldimethyl-silyloxy-13-thia-15α-hydroxy-16,17,18,19,20- pentanor-15-p-bromphenyl-
6,9-Dioxo-11α-tert.-butyldimethyl-silyloxy-13-thia-15β-hydroxy-16,17,18,19-20- pentanor-15-p-bromphenyl-
6,9-Dioxo-11α-tert.-butyldimethyl-silyloxy- 13-thia-15-hydroxy-16,17,18,19,20- pentanor-15-p-tolyl-, Isomerengemisch
6,9-Dioxo-11α-tert.-butyldimethyl-silyloxy- 13-thia-15α-hydroxy-16,17,18,19,20- pentanor-15-p-tolyl-
6,9-Dioxo-11α-tert.-butyldimethyl-silyloxy- 13-thia-15β-hydroxy-16,17,18,19,20- pentanor-15-p-tolyl-
6,9-Dioxo-11α-tert.-butyldimethyl-silyloxy- 13-thia-15-hydroxy-16,17,18,19,20- pentanor-15-o-methoxyphenyl, Isomerengemisch, F. 122°
6,9-Dioxo-11α-tert.-butyldimethyl-silyloxy- 13-thia-15α-hydroxy- 16,17,18,19,20- pentanor-15-o-methoxyphenyl, F. 135°
6,9-Dioxo-11α-tert.-butyldimethyl-silyloxy- 13-thia-15β-hydroxy-16,17,18,19,20- pentanor-15-o-methoxyphenyl, F. 103°
6,9-Dioxo-11α-tert.-butyldimethyl-silyloxy- 13-thia-15- hydroxy-16,17,18,19,20- pentanor-15-(2-pyridyl)-, Isomerengemisch, F. 117°
6,9-Dioxo-11α-tert.-butyldimethyl-silyloxy-13-thia-15α-hydroxy-16,17,18,19,20- pentanor-15-(2-pyridyl)-
6,9-Dioxo-11α-tert.-butyldimethyl-silyloxy- 13-thia-15β-hydroxy-16,17,18,19,20- pentanor-15-(6-pyridyl)-
6,9-Dioxo-11α-tert.-butyldimethyl-silyloxy- 13-thia-15-hydroxy-16- methyl-18-prostinsäure-p-benzamidophenylester, Isomerengemisch, F. 120–121°
6-Oxo-11-O-tert.-butyldimethylsilyl-13-thia-13,14-dihydro-16,17,18,19,20-pentanor-15-phenyl-$PGE_1$-p- benzamidophenylester, F. 126°
(8,11,12-ent)-6-Oxo-11-O-tert.-butyldimethylsilyl- 13-thia- 13,14-dihydro- 16,17,18,19,20-pentanor-15-phenyl-$PGE_1$-p-benz-amidophenylester, F. 132°.

Beispiel 2

Analog Beispiel 1 erhält man aus 2-Hydroxy-3-m-chlorphenoxy-propanthiol und 6-Oxo-7-(3-hydroxy-5-oxo-1- cyclopentenyl)- heptansäure-p-benzamidophenylester den 6,9-Dioxo-11α,15-dihydroxy- 13-thia-17,18,19,20- tetranor- 16-m-chlorphenoxyprostansäure- p-benzamidophenylester, F. 77°.

Analog sind erhältlich:
6,9-Dioxo-11α,15- dihydroxy-13-thia-16,17,18,19,20- pentanor-15-o-chlorphenyl-prostansäure- p-benzamido-phenylester, Isomerengemisch
6,9-Dioxo-11α,15α-dihydroxy-13-thia-16,17,18 19,20-pentanor-15- o-chlorphenyl-prostansäure- p-benzamido-phenylester, F. 62°
6,9-Dioxo-11α,15β-dihydroxy-13- thia-16,17,18,19,20- pentanor-15-o- chlorphenyl-prostansäure- p-benzamido-phenylester, Rf 0,20.

Beispiel 3

Analog Beispiel 1 erhält man aus 2-Hydroxy-2-methylheptanthiol und 6-Oxo-7- (3-tert.-butoxy-5-oxo-1- cyclopentenyl)- heptansäure-p-benzamidophenylester den 6,9-Dioxo-11α-tert.-butoxy-13- thia-15-hydroxy-15- methyl-prostansäure- p-benzamidophenylester als Isomerengemisch.

Analog erhält man die folgenden Prostansäure-p-benzamidophenylester:
6,9-Dioxo-11α-tert.butoxy-13-thia-15- hydroxy-, Isomerengemisch,
6,9-Dioxo-11α-tert.-butoxy-13-thia-15α-hydroxy-
6,9-Dioxo-11α-tert.-butoxy-13-thia-15β-hydroxy-
6,9-Dioxo-11α-tert.-butoxy-13-thia-15-hydroxy-20- methyl-, Isomerengemisch
6,9-Dioxo-11α-tert.-butoxy-13-thia-15α-hydroxy-20-methyl-
6,9-Dioxo-11α-tert.-butoxy-13-thia-15β-hydroxy-20-methyl-
6,9-Dioxo-11α-tert.-butoxy-13-thia-15-hydroxy- 16-methyl-, Isomerengemisch
6,9-Dioxo-11α-tert.-butoxy-13-thia-15α-hydroxy-16-methyl-
6,9-Dioxo-11α-tert.-butoxy-13- thia-15β-hydroxy- 16-methyl-
6,9-Dioxo-11α-tert.-butoxy-13-thia-15-hydroxy- 16,20-dimethyl-, Isomerengemisch
6,9-Dioxo-11α-tert.-butoxy-13-thia-15α-hydroxy- 16,20-dimethyl-
6,9-Dioxo-11α-tert.-butoxy-13-thia-15β-hydroxy- 16,20-dimethyl-
6,9-Dioxo-11α-tert.-butoxy-13- thia-15-hydroxy-17,18,19,20- tetranor-16-m-chlorphenoxy-, Isomerengemisch,
6,9-Dioxo-11α-tert.-butoxy-13-thia-15-hydroxy- 16,17,18,19,20- pentanor-15-phenyl-, Isomerengemisch
6,9-Dioxo-11α-tert.-butoxy-13-thia- 15α-hydroxy-16,17,18,19,20- pentanor-15-phenyl-
6,9-Dioxo-11α-tert.-butoxy-13-thia-15β- hydroxy-16,17,18,19,20- pentanor-15-phenyl-
6,9-Dioxo-11α-tert.-butoxy-13-thia-15-hydroxy-16,17,18,19,20-15-o-chlorphenyl-, Isomerengemisch
6,9-Dioxo-11α-tert.-butoxy-13-thia-15α- hydroxy-16,17,18,19,20-pentanor-15-o-chlorphenyl-
6,9-Dioxo-11α-tert.-butoxy-13-thia-15β-hydroxy-16,17,18,19,20-pentanor-15-o-chlorphenyl
sowie
6,9-Dioxo-11α-tert.-butoxy-13-thia-15-hydroxy- 16-methyl-18-prostinsäure-p- benzamidophenylster, Isomerengemisch
6-Oxo-11-O-tert.-butyl-13-thia- 13,14-dihydro-16, 17,18,19,20- pentanor-15- phenyl-$PGE_1$- p-benzamidophenylester
(8,11,12-ent)- 6-Oxo-11-O-tert.-butyl-13-thia-13,14-dihydro-16,17,18,19,20- pentanor-15-phenyl- $PGE_1$-p-benzamidophenylester.

Beispiel 4

Analog Beispiel 1 erhält man aus 2-Hydroxy-2-methyl-heptanthiol und 6-Oxo-7- (3-tert.-butoxymethoxy-5- oxo-1-cyclopentenyl)- heptansäure- p-benzamidophenylester den 6,9-Dioxo-11α-tert.-butoxymethoxy- 13-thia-15- hydroxy-15-methyl- prostansäure-p- benzamidophenylester als Isomerengemisch.

Analog erhält man die folgenden Prostansäure-p-benzamidophenylester:

6,9-Dioxo-11α-tert.-butoxymethoxy-13- thia-15-hydroxy-, Isomerengemisch

6,9-Dioxo- 11α-tert.- butoxymethoxy- 13-thia-15α-hydroxy-

6,9-Dioxo- 11α-tert.- butoxymethoxy- 13- thia-15β-hydroxy-

6,9-Dioxo- 11α-tert.- butoxymethoxy- 13-thia-15-hydroxy-20-methyl-, Isomerengemisch

6,9-Dioxo- 11α-tert.- butoxymethoxy- 13- thia-15α-hydroxy-20-methyl-

6,9-Dioxo-11α-tert.-butoxymethoxy-13-thia-15β-hydroxy-20-methyl-

6,9-Dioxo-11α-tert.-butoxymethoxy-13-thia-15-hydroxy-16-methyl-, Isomerengemisch

6,9-Dioxo-11α-tert.-butoxymethoxy-13-thia-15α- hydroxy-16-methyl-

6,9-Dioxo-11α-tert.-butoxymethoxy-13-thia-15β-hydroxy- 16-methyl-

6,9-Dioxo-11α-tert.-butoxymethoxy-13-thia-15-hydroxy- 16,20-dimethyl-, Isomerengemisch

6,9-Dioxo-11α-tert.-butoxymethoxy-13-thia-15α-hydroxy- 16,20-dimethyl-

6,9-Dioxo-11α-tert.-butoxymethoxy-13-thia-15β-hydroxy- 16,20-dimethyl-

6,9-Dioxo-11α-tert.-butoxymethoxy-13-thia-15-hydroxy- 17,18,19,20 -tetranor-16-m-chlorphenoxy-, Isomerengemisch

6,9-Dioxo-11α-tert.-butoxymethoxy-13-thia-15-hydroxy- 16,17,18,19,20-pentanor-15- phenyl-, Isomerengemisch

6,9-Dioxo-11α-tert.-butoxymethoxy-13-thia-15α- hydroxy-16,17,18,19,20-pentanor-15-phenyl-

6,9-Dioxo-11α-tert.-butoxymethoxy-13-thia-15β- hydroxy-16,17,18,19,20 -pentanor-15-phenyl-

6,9-Dioxo-11α-tert.-butoxymethoxy-13-thia-15-hydroxy- 16,17,18,19,20-15- o-chlorphenyl-, Isomerengemisch

6,9-Dioxo-11α-tert.-butoxymethoxy-13-thia-15α- hydroxy-16,17,18,19,20-pentanor-15-o-chlorphenyl-

6,9-Dioxo-11α-tert.-butoxymethoxy-13-thia-15β- hydroxy- 16,17,18,19,20 -pentanor-15-o-chlorphenyl-

sowie

6,9-Dioxo-11α-tert.-butoxymethoxy-13-thia-15-hydroxy-16-methyl-18- prostinsäure-p-benzamidophenylester, Isomerengemisch

6-Oxo-11-O-tert.-butoxymethyl-13-thia-13,14-dihydro-16,17,18,19,20-pentanor-15- phenyl-$PGE_1$-p- benzamidophenylester

(8,11,12-ent)- 6-Oxo-11-O-tert.-butoxymethyl-13-thia- 13,14-dihydro- 16,17,18,19,20-pentanor-15- phenyl-$PGE_1$-p-benzamidophenylester.

Beispiel 5

Analog Beispiel 1 erhält man aus 2-Hydroxy-2-methyl-heptanthiol und 6-Oxo-7-[3-(tetrahydropyranyl- 2-oxy)- 5-oxo- 1-cyclopentenyl]-heptansäure-p-benzamidophenylester den 6,9-Dioxo- 11α-tetrahydropyranyl-2-oxy-13- thia-15-hydroxy-15-methyl- prostansäure-p- benzamidophenylester als Isomerengemisch.

Analog erhält man die folgenden Prostansäure-p- benzamidophenylester:

6,9-Dioxo-11α-(tetrahydropyranyl-2-oxy)- 13-thia-15-hydroxy-, Isomerengemisch

6,9-Dioxo-11α-(tetrahydropyranyl-2-oxy)- 13-thia-15α-hydroxy-

6,9-Dioxo-11α-tert.-butoxy-13-thia- 15β-hydroxy-

6,9-Dioxo-11α-(tetrahydropyranyl-2-oxy)- 13-thia-15-hydroxy- 20-methyl-, Isomerengemisch

6,9-Dioxo-11α-(tetrahydropyranyl-2-oxy)- 13-thia-15α- hydroxy-20-methyl-

6,9-Dioxo-11α-(tetrahydropyranyl-2-oxy)- 13-thia-15β-hydroxy-20-methyl-

6,9-Dioxo-11α-(tetrahydropyranyl-2-oxy)- 13-thia-15-hydroxy16-methyl-, Isomerengemisch

6,9-Dioxo-11α-(tetrahydropyranyl-2-oxy)- 13-thia-15α-hydroxy-16-methyl-

6,9-Dioxo-11α-(tetrahydropyranyl-2-oxy)- 13-thia-15β-hydroxy-16-methyl-

6,9-Dioxo-11α-(tetrahydropyranyl-2-oxy)- 13-thia-15-hydroxy-16,20-dimethyl-, Isomerengemisch

6,9-Dioxo-11α-(tetrahydropyranyl-2-oxy)- 13-thia-15α-hydroxy-16,20-dimethyl-

6,9-Dioxo-11α-(tetrahydropyranyl-2-oxy)- 13-thia-15β-hydroxy-16,29-dimethyl-

6,9-Dioxo-11α-(tetrahydropyranyl-2-oxy)- 13-thia-15-hydroxy-17,18,19,20- tetranor-16-m-chlorphenoxy-, Isomerengemisch

6,9-Dioxo-11α-(tetrahydropyranyl-2-oxy)- 13-thia-15-hydroxy- 16,17,18,19,20-pentanor-15-phenyl-, Isomerengemisch

6,9-Dioxo-11α-(tetrahydropyranyl-2-oxy)- 13-thia-15α-hydroxy-16,17,18,19,20-pentanor-15-phenyl-

6,9-Dioxo-11α-(tetrahydropyranyl-2-oxy)- 13-thia-15β-hydroxy-16,17,18,19,20-pentanor-15-phenyl-

6,9-Dioxo-11α-(tetrahydropyranyl-2-oxy)- 13-thia-15-hydroxy- 16,17,18,19,20-pentanor-15-o-chlorphenyl-, Isomerengemisch,

6,9-Dioxo-11α-(tetrahydropyranyl-2-oxy)- 13-thia-15α-hydroxy-16,17,18,19,20-pentanor-15-o-chlorphenyl-

6,9-Dioxo-11α-(tetrahydropyranyl-2-oxy)- 13-thia-15β-hydroxy-16,17,18,19,20- pentanor-15-o-chlorphenyl-

sowie

6,9-Dioxo-11α-(tetrahydropyranyl-2-oxy)- 13-thia-15-hydroxy-16-methyl-18- prostinsäure-p-benzamidophenylester, Isomerengemisch

6-Oxo-11-O- (tetrahydropyran-2-yl)- 13-thia-13,14-dihydro-16,17,18,19,20-pentanor-15-phenyl-$PGE_1$-p- benzamidophenyl-ester

(8,11,12-ent)-6-Oxo-11-O- (tetrahydropyran-2-yl)- 13-thia-13,14-dihydro-16,17,18,19,20-pentanor-15-phenyl-$PGE_1$-p-benzamido-phenylester.

Beispiel 6

Analog Beispiel 1 erhält man aus 2-Hydroxy-2-methylheptanthiol und 6-Oxo-7- (3-acetoxy-5-oxo-1-cyclopentenyl)- heptansäure-p- benzamidophenylester den 6,9-Dioxo-11α-acetoxy-13-thia-15- hydroxy-15-methyl- prostansäure-p-benzamidophenylester als Isomerengemisch.

Analog erhält man die folgenden Prostansäure-p-benzamidophenylester:

6,9-Dioxo-11α-acetoxy-13-thia-15-hydroxy-, Isomerengemisch
6,9-Dioxo-11α-acetoxy-13-thia-15α-hydroxy-
6,9-Dioxo-11α-acetoxy-13-thia-15β-hydroxy-
6,9-Dioxo-11α-acetoxy-13-thia-15-hydroxy-20-methyl-, Isomerengemisch
6,9-Dioxo-11α-acetoxy-13-thia-15α-hydroxy-20-methyl-
6,9-Dioxo-11α-acetoxy-13-thia-15β-hydroxy-20-methyl-
6,9-Dioxo-11α-acetoxy-13-thia-15-hydroxy-16-methyl-, Isomerengemisch
6,9-Dioxo-11α-acetoxy-13-thia-15α-hydroxy-16-methyl-
6,9-Dioxo-11α-acetoxy-13-thia-15β-hydroxy-16-methyl-
6,9-Dioxo-11α-acetoxy-13-thia-15-hydroxy-16,20-dimetyl-, Isomerengemisch
6,9-Dioxo-11α-acetoxy-13-thia-15α-hydroxy-16,20-dimethyl-
6,9-Dioxo-11α-acetoxy-13-thia-15β-hydroxy-16,20-dimethyl-
6,9-Dioxo-11α-acetoxy-13-thia-15-hydroxy-17,18,19,20-tetranor-16-m-chlorphenoxy-, Isomerengemisch
6,9-Dioxo-11α-acetoxy-13-thia-15-hydroxy-16,17,18,19,20-pentanor-15-phenyl-, Isomerengemisch
6,9-Dioxo-11α-acetoxy-13-thia-15α- hydroxy-16,17,18,19,20- pentanor-15-phenyl-
6,9-Dioxo-11α-acetoxy-13-thia-15β-hydroxy-16,17,18,19,20-pentanor- 15-phenyl-, Isomerengemisch
6,9-Dioxo-11α-acetoxy-13-thia-15- hydroxy-16,17,18,19,20-pentanor-15-o-chlorphenyl, Isomerengemisch
6,9-Dioxo-11α-acetoxy-13-thia-15α-hydroxy-16,17,18,19,20- pentanor-15-o-chlorphenyl-
6,9-Dioxo-11α-acetoxy-13-thia-15β-hydroxy-16,17,18,19,20-pentanor-15- o-chlorphenyl-
sowie
6,9-Dioxo-11α-acetoxy-13-thia-15-hydroxy-16-methyl-18-prostinsäure-p- benzamidophenylester, Isomerengemisch
6-Oxo-11-O-acetyl-13-thia-13,14-dihydro-16,-17,18,19,20-pentanor-15-phenyl- $PGE_1$-p-benzamidophenyl-ester
(8,11,12-ent)-6-Oxo-11-O-acetyl-13-thia-13,14-dihydro-16,17,18,19,20- pentanor-15-phenyl-$PGE_1$ p-benzamidophenylester.

Beispiel 7

Analog Beispiel 1 erhält man aus 2-Hydroxy-2-methyl-heptanthiol und 6-Oxo-7- (3-trimethylacetoxy-5-oxo-1- cyclopen-tenyl)- heptansäure-p- benzamidophenylester den 6,9-Dioxo-11α-trimethylacetoxy- 13-thia-15- hydroxy-15-methyl-prostansäure-p-benzamidophenylester als Isomerengemisch.

Analog erhält man die folgenden Prostansäure-p-benzamidophenylester:

6,9-Dioxo-11α-trimethylacetoxy-13-thia-15-hydroxy-, Isomerengemisch
6,9-Dioxo-11α-trimethylacetoxy-13-thia-15α-hydroxy-
6,9-Dioxo-11α-trimethylacetoxy-13-thia-15β-hydroxy-
6,9-Dioxo-11α-trimethylacetoxy-13-thia- 15-hydroxy-20-methyl-, Isomerengemisch
6,9-Dioxo-11α-trimethylacetoxy-13-thia-15α-hydroxy-20-methyl-
6,9-Dioxo-11α-trimethylacetoxy-13-thia-15β-hydroxy-20-methyl-
6,9-Dioxo-11α-trimethylacetoxy-13-thia-15-hydroxy-16-methyl-, Isomerengemisch
6,9-Dioxo-11α-trimethylacetoxy-13-thia-15α-hydroxy-16-methyl-
6,9-Dioxo-11α-trimethylacetoxy-13-thia-15β-hydroxy-16-methyl-
6,9-Dioxo-11α-trimethylacetoxy-13-thia- 15-hydroxy-16,20-dimethyl-, Isomerengemisch
6,9-Dioxo-11α-trimethylacetoxy-13-thia-15α-hydroxy-16,20-dimethyl-
6,9-Dioxo-11α-trimethylacetoxy-13-thia-15β-hydroxy- 16,20-dimethyl-
6,9-Dioxo-11α-trimethylacetoxy-13-thia-15-hydroxy- 17,18,19,20- tetranor-16-m-chlorphenoxy-, Isomerengemisch
6,9-Dioxo-11α-trimethylacetoxy-13-thia-15-hydroxy- 16,17,18,19,20- pentanor- 15-phenyl-, Isomerengemisch
6,9-Dioxo-11α-trimethylacetoxy-13-thia-15α-hydroxy- 16,17,18,29,20- pentanor-15-phenyl-,
6,9-Dioxo-11α-trimethylacetoxy-13-thia-15β-hydroxy-16,17,18,19,20- pentanor-15-phenyl-,
6,9-Dioxo-11α-trimethylacetoxy-13-thia-15-hydroxy- 16,17,18,19,20- pentanor-15-o-chlorphenyl-, Isomerengemisch
6,9-Dioxo-11α-trimethylacetoxy-13-thia-15α-hydroxy- 16,17,18,19,20- pentanor-15-o-chlorphenyl-
6,9-Dioxo-11α-trimethylacetoxy-13-thia-15β-hydroxy- 16,17,18,19,20 -pentanor- 15-o-chlorphenyl-
sowie
6,9-Dioxo-11α-trimethylacetoxy-13-thia-15-hydroxy- 16-methyl- 18-prostinsäure-p- benzamidophenylester, Isomerengemisch
6-Oxo-11-O-trimethylacetoyl-13-thia- 13,14-dihydro-16,17,18,19,20- pentanor-15-phenyl-$PGE_1$- p-benzamidophenylester
(8,11,12-ent)-6-Oxo-11-O-trimethylacetyl-13-thia-13,14-dihydro-16,17,18,19,20- pentanor-15-phenyl-$PGE_1$-p- benzamidophenylester.

Beispiel 8

Analog Beispiel 1 erhält man aus 2-Hydroxy-2-methylheptanthiol und 6-Oxo-7-(3-tert.-butylacetoxy-5- oxo-1-cyclopen-tenyl)- heptansäure-p-benzamidophenylester den 6,9-Dioxo-11α-tert.-butylacetoxy- 13-thia-15-hydroxy-15-methyl- prostansäure-p- benzamidophenylester als Isomerengemisch.

Analog erhält man die folgenden Prostansäure-p-benzamidophenylester:

6,9-Dioxo-11α-tert.butylacetoxy-13-thia- 15-hydroxy-, Isomerengemisch
6,9-Dioxo-11α-tert.butylacetoxy-13-thia- 15α-hydroxy-
6,9-Dioxo-11α-tert.butylacetoxy-13-thia- 15β-hydroxy-
6,9-Dioxo-11α-tert.butylacetoxy-13-thia-15-hydroxy-20- methyl-, Isomerengemisch
6,9-Dioxo-11α-tert.butylacetoxy-13-thia-15α-hydroxy-20-methyl-
6,9-Dioxo-11α-tert.butylacetoxy-13- thia-15β-hydroxy-20-methyl-
6,9-Dioxo-11α-tert.butylacetoxy-13-thia-15-hydroxy- 16-methyl-, Isomerengemisch
6,9-Dioxo-11α-tert.butylacetoxy-13-thia-15α-hydroxy-16-methyl-
6,9-Dioxo-11α-tert.butylacetoxy-13-thia-15β-hydroxy-16-methyl-
6,9-Dioxo-11α-tert.butylacetoxy-13-thia-15-hydroxy-16,20-dimethyl-, Isomerengemisch
6,9-Dioxo-11α-tert.butylacetoxy-13-thia-15α-hydroxy-16,20-dimethyl-
6,9-Dioxo-11α-tert.butylacetoxy-13-thia-15β-hydroxy-16,20-di-methyl-
6,9-Dioxo-11α-tert.butylacetoxy-13-thia-15-hydroxy-16,17,18,19,20-pentanor-15-phenyl-, Isomerengemisch
6,9-Dioxo-11α-tert.butylacetoxy-13- thia-15α-hydroxy- 16,17,18,19,20-pentanor-15-phenyl-,
6,9-Dioxo-11α-tert.butylacetoxy-13-thia-15β-hydroxy- 16,17,18,19,20-pentanor-15-phenyl-,
6,9-Dioxo-11α-tert.butylacetoxy-13-thia-15-hydroxy-16,17,18,19,20-pentanor-15-o-chlorphenyl-, Isomerengemisch
6,9-Dioxo-11α-tert.butylacetoxy-13-thia- 15-hydroxy-17,18,19,20- tetranor-16-m-chlorphenoxy-, Isomerengemisch
6,9-Dioxo-11α-tert.butylacetoxy-13-thia-15α-hydroxy-16,17,18,19,20-pentanor-15-o-chlorphenyl-
6,9-Dioxo-11α-tert.butylacetoxy-13-thia-15β-hydroxy-16,17,18,19,20-pentanor-15-o-chlorphenyl-
sowie
6,9-Dioxo-11α-tert.butylacetoxy-13-thia-15-hydroxy-16- methyl-18-prostinsäure-p- benzamidophenylester, Isomerengemisch
6-Oxo-11-O-tert.-butylacetyl-13-thia-13,14-dihydro-16,17,18,19,20-pentanor-15-phenyl-$PGE_1$-p- benzamidophenylester
(8,11,12-ent)-6-Oxo-11-O-tert.-butylacetyl-13-thia-13,14- dihydro-16,17,18,19,20-pentanor- 15-phenyl-$PGE_1$-p-benzamidophenylester.

Beispiel 9

Eine Lösung von 24 g 2-Hydroxy-2-methyl-heptanthiol und 84 g Diisopropylamin in 300 ml THF wird 2 Std. gekocht, dann auf −25° abgekühlt und mit einer vorgekühlten Lösung von 24 g 6-Oxo-7- (3-hydroxy-5-oxo-1-cyclopentenyl)- heptansäure in 75 ml THF versetzt. Man arbeitet weiter wie in Beispiel 1 angegeben und erhält ölige 6,9-Dioxo- 11α,15-dihydroxy-13-thia-15- methyl-prostansäure.

Analog erhält man:

6,9-Dioxo-11α,15-dihydroxy-13-thia-15-methyl- prostansäuremethylester,
6,9-Dioxo-11α,15-dihydroxy-13-thia-15- methyl-prostansäurebutylester
6,9-Dioxo-11α,15-dihydroxy-13- thia-15-methyl- prostansäurephenylester
6,9-Dioxo-11α,15-dihydroxy-13- thia-15-methyl-prostansäure-p- biphenylester
6,9-Dioxo-11α,15-dihydroxy-13-thia-16- methyl-18-prostensäure
6,9-Dioxo-11α,15-dihydroxy-13-thia-15,16- dimethyl-prostansäure
6,9-Dioxo-11α,15-dihydroxy-13-thia-16,16- dimethyl-prostansäure
6,9-Dioxo-11α,15-dihydroxy-13-thia-17- phenyl-18,19,20-trinor-prostansäure
6,9-Dioxo-11α,15-dihydroxy-13-thia-20- fluor-prostansäure
6,9-Dioxo-11α,15-dihydroxy-13-thia-16- cyclopentyl-17,18,19,20-tetranor-prostansäure
6,9-Dioxo-11α,15-dihydroxy-13-thia-16- cyclohexyl- 17,18,19,20-tetranor-prostansäure
6,9-Dioxo-11α,15-dihydroxy-13-thia-16-(4-methylcyclohexyl)- 17,18,19,20-tetranor-prostansäure
6,9-Dioxo-11α,15-dihydroxy-13-thia-17-p-fluorphenyl-18,19,20-trinor-prostansäure
6,9-Dioxo-11α,15-dihydroxy-13-thia-17- p-bromphenyl-18,19,20-trinor- prostansäure
6,9-Dioxo-11α,15-dihydroxy-13-thia-17-p- tolyl-18,19,20- trinor-prostansäure
6,9-Dioxo-11α,15-dihydroxy-13-thia-17- p-hydroxyphenyl-18,19,20-trinor-prostansäure
6,9-Dioxo-11α,15-dihydroxy-13-thia-17- p-methoxyphenyl-18,19,20-trinor-prostansäure
6,9-Dioxo-11α,15-dihydroxy-13-thia-17-m-trifluormethylphenyl- 18,19,20-trinor-prostansäure
6,9-Dioxo-11α,15-dihydroxy-13-thia-17-(4-pyridyl)- 18,19,20-trinor-prostansäure
6,9-Dioxo-11α,15-dihydroxy-13-thia-17-(1-naphthyl)- 18,19,20-trinor-prostansäure
6,9-Dioxo-11α,15-dihydroxy-13-thia-17-(2-thienyl)- 18,19,20-trinor-prostansäure
6,9-Dioxo-11α,15-dihydroxy-13-thia-17-methoxy-18,19,20-trinor-prostansäure
6,9-Dioxo-11α,15-dihydroxy-13-thia-17-methylthio- 18,19,20-trinor-prostansäure
6,9-Dioxo-11α,15-dihydroxy-13-thia-16- phenoxy- 18,19,20-trinor-prostansäure
6,9-Dioxo-11α,15-dihydroxy-13-thia-16- p-fluorphenoxy-18,19,20-trinor-prostansäure
6,9-Dioxo-11α,15-dihydroxy-13-thia-16-(3,4,5-trimethoxyphenoxy)- 18,19,29-trinor-prostansäure.

Beispiel 10

Man löst 10 g 6,9-Dioxo-11α-tert.butyldimethylsilyloxy-13- thia-15-hydroxy-15- methyl-prostansäure-p- benzamidophenylester in einem

Gemisch aus 43 ml 40%iger Flusssäure und 243 ml Acetonitril, rührt 2,5 Std. bei 20°, gibt $NaHCO_3$ bis pH 8 hinzu und arbeitet wie üblich auf. Man erhält 6,9-Dioxo-11α,15-dihydroxy-13-thia-15-methylprostansäure- p-benzamidophenylester, Isomerengemisch, F. 69–70 °C.

Analog sind durch Hydrolyse der entsprechenden 3α-tert.-Butyldimethyl-silylether die folgenden Prostansäure-p-benzamidophenylester erhältlich:

6,9-Dioxo-11α,15α-dihydroxy-13-thia-15-methyl-
6,9-Dioxo-11α,15β-dihydroxy-13- thia-15-methyl-
6,9-Dioxo-11α,15-dihydroxy-13-thia, Isomerengemisch
6,9-Dioxo-11α,15α-dihydroxy-13-thia-, F. 94°
6,9-Dioxo-11α,15β-dihydroxy-13-thia-, F. 113°
6,9-Dioxo-11α,15-dihydroxy-13-thia-20-methyl-, Isomerengemisch
6,9-Dioxo11α,15α-dihydroxy-13-thia- 20-methyl-, F. 93°
6,9-Dioxo-11α,15β-dihydroxy-13-thia-20-methyl-, F. 113°
6,9-Dioxo-11α,15-dihydroxy-13-thia-16- methyl-, Isomerengemisch, F. 74°
6,9-Dioxo-11α,15α-dihydroxy-13-thia-16-methyl-, F. 99°
6,9-Dioxo-11α,15β-dihydroxy-13-thia-16-methyl-, F. 114°
6,9-Dioxo-11α,15-dihydroxy-13-thia-16,20-dimethyl-, Isomerengemisch, F. 76°
6,9-Dioxo-11α,15α-dihydroxy-13-thia-16,20-dimethyl-, F. 69–70°
6,9-Dioxo-11α,15β-dihydroxy-13-thia-16,20-dimethyl-, F. 81–82°
6,9-Dioxo-11α,15-dihydroxy-13-thia-20-ethyl-, Isomerengemisch, F. 64°
6,9-Dioxo-11α,15α-dihydroxy-13-thia-20-ethyl-, F. 63°
6,9-Dioxo-11α,15β-dihydroxy-13-thia-20-ethyl-, F. 79°
6,9-Dioxo-11α,15-dihydroxy-13-thia-15,20- dimethyl-, Isomerengemisch, F. 71°
6,9-Dioxo-11α,15α-dihydroxy-13-thia-15,20-dimethyl-
6,9-Dioxo-11α,15β-dihydroxy-13-thia- 15,20-dimethyl-
6,9-Dioxo-11α,15-dihydroxy-13-thia-16,16,21-trimethyl-, Isomerengemisch
6,9-Dioxo-11α,15α-dihydroxy-13-thia-16,16,21-trimethyl-
6,9-Dioxo-11α,15β-dihydroxy-13-thia-16,16,21-trimethyl-
6,9-Dioxo-11α,15-dihydroxy-13-thia-15-methyl- 20-ethyl-, Isomerengemisch, F. 73°
6,9-Dioxo-11α,15α-dihydroxy-13-thia-15-methyl-20- ethyl-
6,9-Dioxo-11α,15β-dihydroxy-13-thia-15-methyl- 20-ethyl-
6,9-Dioxo-11α,15-dihydroxy-13-thia-16-methyl- 20-ethyl-, Isomerengemisch, Rf 0,49–0,52.
6,9-Dioxo-11α,15α-dihydroxy-13-thia-16-methyl- 20-ethyl-, Rf 0,52
6,9-Dioxo-11α,15β-dihydroxy-13-thia-16-methyl- 20-ethyl-, F. 87°; Rf 0,49
6,9-Dioxo-11α,15-dihydroxy-13-thia-16,17,-18,19,20-pentanor- 15-cyclohexyl-, Isomerengemisch, F. 83°
6,9-Dioxo-11α,15α-dihydroxy-13-thia-16,17,-18,19,20- pentanor-15 -cyclohexyl-, F. 78°
6,9-Dioxo-11α,15β-dihydroxy-13-thia-16,17,18,19,20-pentanor-15- cyclohexyl-, F. 90°
6,9-Dioxo-11α,15-dihydroxy-13-thia-18,19,20-trinor-17-cyclohexyl-, Isomerengemisch, Rf 0,45–0,5
6,9-Dioxo-11α,15α-dihydroxy-13-thia-18,19,-20- trinor-17-cyclohexyl-, Rf 0,5
6,9-Dioxo-11α,15β-dihydroxy-13-thia-18,19,-20- trinor-17-cyclohexyl-, Rf 0,45
6,9-Dioxo-11α,15-dihydroxy-13-thia-16,17,-18,19,20- pentanor-15-phenyl-, Isomerengemisch, Rf 0,40–0,45
6,9-Dioxo-11α,15α-dihydroxy-13-thia-16,17,-18,19,20- pentanor-15-phenyl-, Rf 0,45
6,9-Dioxo-11α,15β-dihydroxy-13-thia-16,17,-18,19,20- pentanor-15-phenyl-, F. 125°, Rf. 0,40
6,9-Dioxo-11α,15-dihydroxy-13-thia-18,19,20-trinor-16- phenyl-, Isomerengemisch, Rf 0,40–0,45
6,9-Dioxo-11α,15α-dihydroxy-13-thia-18,19,20-trinor-16- phenyl-, Rf 0,45
6,9-Dioxo-11α,15β-dihydroxy-13-thia-18,19,20-trinor-16- phenyl, Rf. 0,40
6,9-Dioxo-11α,15-dihydroxy-13-thia-16-methyl- 18,19,20-trinor-17-phenyl-, Isomerengemisch
6,9-Dioxo-11α,15α-dihydroxy-13-thia-16-methyl- 18,19,20-trinor-17-phenyl-, F. 130°, Rf 0,4
6,9-Dioxo-11α,15β-dihydroxy-13-thia-16-methyl- 18,19,20-trinor-17-phenyl-, Rf 0,3
6,9-Dioxo-11α,15-dihydroxy-13-thia-16,16- dimethyl- 18,19,20-trinor-17-phenyl-, Isomerengemisch
6,9-Dioxo-11α,15α-dihydroxy-13-thia-16,16-dimethyl-18,19,20-trinor-17-phenyl-
6,9-Dioxo-11α,15β-dihydroxy-13-thia-16,16-dimethyl-18,19,20- trinor-17-phenyl-
6,9-Dioxo-11α,15-dihydroxy-13-thia-16,17,18, 19,20- pentanor-15-p-fluorphenyl-, Isomerengemisch, Rf 0,42–0,45
6,9-Dioxo-11α,15α-dihydroxy-13-thia-16,17, 18,19,20- pentanor-15-p-fluorphenyl-, Rf 0,45
6,9-Dioxo-11α,15β-dihydroxy-13-thia-16,17, 18,19,20- pentanor-15-p-fluorphenyl-, Rf 0,42
6,9-Dioxo-11α,15-dihydroxy-13-thia-16,17, 18,19,20- pentanor-15-o-chlorphenyl-,Isomerengemisch, Rf 0,08–0,12 ($CH_2Cl_2/CH_3OH$ 97:3)
6,9-Dioxo-11α,15α-dihydroxy-13-thia-16,17, 18,19,20- pentanor-15-o-chlorphenyl-, F. 62°, Rf 0,12 ($CH_2Cl_2/CH_3OH$ 97:3)
6,9-Dioxo-11α,15β-dihydroxy-13-thia-16,17, 18,19,20- pentanor-15-o-chlorphenyl-, Rf 0,08 ($CH_2Cl_2/CH_3OH$ 97:3)
6,9-Dioxo-11α,15-dihydroxy-13-thia-16,17, 18,19,20- pentanor-15-m-chlorphenyl-, Isomerengemisch, Rf 0,5–0,55 (Toluol/Aceton 3:2)
6,9-Dioxo-11α,15α-dihydroxy-13-thia-16,17,

18,9,20-pentanor-15-m-chlorphenyl-, F. 113°, Rf 0,55 (Toluol/Aceton 3:2)
6,9-Dioxo-11α,15β-dihydroxy-13-thia-16,17,18,19,20-pentanor-15-m-chlorphenyl-, Rf 0,5 (Toluol/Aceton 3:2)
6,9-Dioxo-11α,15-dihydroxy-13-thia-16,17,18,19,20-pentanor-15-p-chlorphenyl-, Isomerengemisch
6,9-Dioxo-11α,15α-dihydroxy-13-thia-16,17,18,19,20-pentanor-15-p-chlorphenyl-, F. 18°
6,9-Dioxo-11α,15β-dihydroxy-13-thia-16,17,18,19,20-pentanor-15-p-chlorphenyl-, F. 18°
6,9-Dioxo-11α,15-dihydroxy-13-thia-16,17,18,19,20-pentanor-15-p-bromphenyl-, Isomerengemisch, Rf 0,42–0,47
6,9-Dioxo-11α,15α-dihydroxy-13-thia-16,17,18,19,20-pentanor-15-p-bromphenyl, Rf 0,47
6,9-Dioxo-11α,15β-dihydroxy-13-thia-16,17,18,19,20-pentanor-15-p-bromphenyl, F. 142°, Rf. 0,42
6,9-Dioxo-11α,15-dihydroxy-13-thia-16,17,18,19,20-pentanor-15-p-tolyl-, Isomerengemisch
6,9-Dioxo-11α,15α-dihydroxy-13-thia-16,17,18,19,20-pentanor-15-p-toluol-, F. 94°
6,9-Dioxo-11α,15β-dihydroxy-13-thia-16,17,18,19,20-pentanor-15-p-tolyl-, F. 89°
6,9-Dioxo-11α,15-dihydroxy-13-thia-16,17,18,19,20-pentanor-15-o-methoxyphenyl, Isomerengemisch, F. 83°
6,9-Dioxo-11α,15α-dihydroxy-13-thia-16,17,18,19,20-pentanor-15-omethoxyphenyl, F. 85°
6,9-Dioxo-11α,15β-dihydroxy-13-thia-16,17,18,19,20-pentanor-15-o-methoxyphenyl, F. 85°
6,9-Dioxo-11α,15-dihydroxy-13-thia-16,17,18,19,20-pentanor-15-(2-pyridyl)-, Isomerengenmisch, Rf 0,2 ($CH_2Cl_2/CH_3OH$ 94:6)
6,9-Dioxo-11α,15α-dihydroxy-13-thia-16,17,18,19,20-pentanor-15-(2-pyridyl)-
6,9-Dioxo-11α,15β-dihydroxy-13-thia-16,17,18,19,20-pentanor-15-(2-pyridyl)-
sowie
6,9-Dioxo-11α,15-dihydroxy-13-thia-16-methyl-18-prostinsäure-p-benzamidophenylester, Isomerengemisch
6-Oxo-13-thia-13,14-dihydro-16,17,18,19,20-pentanor-15-phenyl-PGE$_1$-p-benzamidophenylester
(8,11,12-ent)-6-Oxo-13-thia-13,14-dihydro-16,17,18,19,20-pentanor-15-phenyl-PGE$_1$-p-benzamidophenylester.

Beispiel 11

Man rührt 1 g 6,9-Dioxo-11α,15-dihydroxy-13-thia-15-methyl-prostansäure-p-benzamidophenylester mit 1 ml Pankreas-Lipase (E. Merck) in 100 ml Wasser 24 Std. bei 20°, chromatographiert an RP-Kieselgel mit Acetonitril/Wasser 1:1 und erhält ölige 6,9-Dioxo-11α,15-dihydroxy-13-thia-15-methyl-prostansäure.

Analog erhält man durch enzymatische Hydrolyse der entsprechenden p-Benzamidophenylester:
6,9-Dioxo-11α,15-dihydroxy-13-thia-prostansäure, Isomerengemisch
6,9-Dioxo-11α,15α-dihydroxy-13-thia-prostansäure
6,9-Dioxo-11α,15β-dihydroxy-13-thia-prostansäure
6,9-Dioxo-11α,15-dihydroxy-13-thia-20-methyl-prostansäure, Isomerengemisch
6,9-Dioxo-11α,15α-dihydroxy-13-thia-20-methyl-prostansäure
6,9-Dioxo-11α,15β-dihydroxy-13-thia-20-methyl-prostansäure
6,9-Dioxo-11α,15-dihydroxy-13-thia-16-methyl-prostansäure, Isomerengemisch
6,9-Dioxo-11α,15α-dihydroxy-13-thia-16-methyl-prostansäure
6,9-Dioxo-11α,15β-dihydroxy-13-thia-16-methyl-prostansäure
6,9-Dioxo-11α,15-dihydroxy-13-thia-16,20-dimethyl-prostansäure, Isomerengemisch
6,9-Dioxo-11α,15α-dihydroxy-13-thia-16,20-dimethyl-prostansäure
6,9-Dioxo-11α,15β-dihydroxy-13-thia-16,20-dimethyl-prostansäure
6,9-Dioxo-11α,15-dihydroxy-13-thia-17,18,-19,20-tetranor-16-m-chlorphenoxy-prostansäure, Isomerengemisch
6,9-Dioxo-11α,15α-dihydroxy-13-thia-17,18,-19,20-tetranor-16-m-chlorphenoxy-prostansäure
6,9-Dioxo-11α,15β-dihydroxy-13-thia-17,18,19,20-tetranor-16-m-chlorphenoxy-prostansäure
6,9-Dioxo-11α,15-dihydroxy-13-thia-16,17,-18,19,20-pentanor-15-o-chlorphenyl-prostansäure, Isomerengemisch
6,9-Dioxo-11α,15α-dihydroxy-13-thia-16,17,18,19,20-pentanor-15-o-chlorphenyl-prostansäure,
6,9-Dioxo-11α,15β-dihydroxy-13-thia-16,17,-18,19,20-pentanor-15-o-chlorphenyl-prostansäure
sowie
6,9-Dioxo-11α,15-dihydroxy-13-thia-16-methyl-18-prostinsäure
6-Oxo-13-thia-13,14-dihydro-16,17,18,19,20-pentanor-15-phenyl-PGE$_1$
(8,11,12-ent)-6-oxo-13-thia-13,14-dihydro-16,17,18,19,20-pentanor-15-phenyl-PGE$^1$.

Beispiel 12

Ein Gemisch von 5,85 g 6,9-Dioxo-11α,15-dihydroxy-13-thia-15-methylprostansäure-p-benzamidophenylester, 2,3 g tert.-Butyl-dimethylchlorsilan, 2,1 g Imidazol und 300 ml DMF wird 16 Std. bei 20° gerührt und wie üblich aufgearbeitet. Man erhält 6,9-Dioxo-11α-tert.-butyldimethylsilyloxy-13-thia-15-hydroxy-methylprostansäure-p-benzamidophenylester, Isomerengemisch, F. 112°.

Beispiel 13

Ein Gemisch von 1 g 6,9-Dioxo-11α,15-dihydroxy-13-thia-15-methyl-prostansäuremethylester, 15 ml Acetanhydrid und 2 Tropfen Pyridin wird 16 Std. bei 20° gerührt und wie üblich aufgearbeitet. Man erhält 6,9-Dioxo-11α-acetoxy-13-thia-15- hydroxy-15-methylprostansäuremethylester, Isomerengemisch.

Beispiel 14

Eine Lösung von 2,09 g 6,9-Dioxo-11α,15-dihydroxy-13- thia-16,20-dimethyl-prostansäure in 30 ml absolutem Methanol wird mit 50 ml 0,1 n Natronlauge versetzt. Die Lösung wird sofort gefriergetrocknet. Man erhält das Natriumsalz der 6,9-Dioxo-11α,15-dihydroxy-13-thia-16,20-dimethyl-prostansäure.

Die nachstehenden Beispiele betreffen Mischungen von Verbindungen der Formel I mit in der Pharmazie üblichen Träger- oder Hilfsstoffen, welche vor allem als Arzneimittel verwendet werden können:

Beispiel A: Tabletten

Ein Gemisch, bestehend aud 3 g 6,9-Dioxo-11α-tert.-butyldimethylsilyloxy- 13-thia-15-hydroxy- 15-methyl-prostansäure-p- benzamidophenylester, 50 g Lactose, 16 g Maisstärke, 2 g Cellulosepulver und 2 g Magnesiumstearat, wird in üblicher Weise zu Tabletten gepresst, derart, dass jede Tablette 1 mg des Wirkstoffes enthält.

Beispiel B: Dragees

Analog Beispiel A werden Tabletten gepresst, die anschliessend in üblicher Weise mit einem Überzug, bestehend aus Zucker, Maisstärke, Talk und Tragant, überzogen werden.

Beispiel C: Injektionslösung

0,1 g 6,9-Dioxo- 11α,15-dihydroxy-13- thia-15- methylprostansäure-p- benzamidophenylester werden in 5 ml Ethanol gelöst, die Lösung wird mit 0,5 l Wasser verdünnt und steril filtriert. Die erhaltene Injektionslösung wird je nach Bedarf in Ampullen enthaltend 2,5 ml, 5 ml oder 10 ml der Injektionslösung gefüllt. Jede Ampulle enthält 0,05, 0,1 oder 0,2 mg Wirkstoff.

Analog sind Tabletten, Dragges und Injektionslösungen erhältlich, die einen oder mehrere der übrigen Wirkstoffe der Formel I enthalten.

**Patentansprüche**

1. Schwefelhaltige 6-Ketoprostaglandine der Formel I

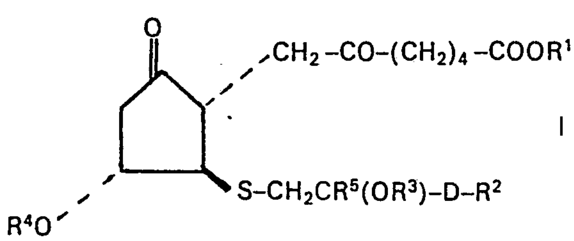

I

worin

D eine Bindung, Alkylen mit 1–3 C-Atomen, cis-Alkenylen mit 2–5 C-Atomen oder Alkinylen mit 2–5 C-Atomen,

$R^1$ H, Alkyl mit 1–4 C-Atomen, Aryl mit 6–12 C-Atomen oder $-C_6H_4NHCOC_6H_5$

$R^2$ Alkyl mit 1–7 C-Atomen, durch Halogen substituiertes Alkyl mit 1–7 C-Atomen, Cycloalkyl mit 5–6 C-Atomen, durch Alkyl mit 1–4 C-Atomen substituiertes Cycloalkyl mit 5–6 C-Atomen, Phenyl, durch F, Cl, Br, Alkyl mit 1–4 C-Atomen, OH, $OCH_3$ oder $CF_3$ substituiertes Phenyl, Pyridyl, Naphthyl, Thienyl, oder, falls D Alkylen mit 1–3 C-Atomen bedeutet, auch Alkoxy mit 1–4 C-Atomen, Alkylthio mit 1–4 C-Atomen, Phenoxy oder durch F, Cl, Br, Alkyl mit 1–4 C-Atomen, OH, $OCH_3$ oder $CF_3$ substituiertes Phenoxy,

$R^3$ und $R^4$ jeweils H, Alkyl mit 1–7 C-Atomen, Tetrahydropyran-2-yl, Trialkylsilyl mit insgesamt 3–12 C-Atomen, Aryl-dialkylsilyl mit insgesamt 8–18 C-Atomen, Alkoxymethyl mit 2–5 C-Atomen, Aryloxymethyl mit 7–11 C-Atomen oder Acyl mit 1–10 C-Atomen und

$R^5$ H oder Alkyl mit 1–3 C-Atomen bedeuten und

....... anzeigt, dass diese Bindung α-ständig ist,

 anzeigt, dass diese Bindung β-ständig ist,

sowie, falls $R^1$ =H ist, deren Salze.

2. a) 6,9-Dioxo- 11α-tert.-butyldimethylsilyloxy- 13-thia- 16,20-dimethyl prostansäure-p-benzamidophenylester

b) 6,9-Dioxo- 11α-tert.-butyldimethylsiloxy-13-thia-15- hydroxy-15- methyl-prostansäurebenzamidophenylester

c) 6,9-Dioxo-11α,15-dihydroxy-13-thia-15-methyl-prostansäure-p- benzamidophenylester.

3. Verfahren zur Herstellung von schwefelhaltigen 6-Ketoprostaglandinen der Formel I nach Anspruch 1, sowie falls $R^1$ =H ist, von deren Salzen, dadurch gekennzeichnet, dass man eine Verbindung der Formel II

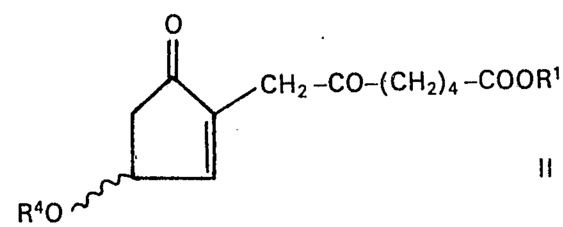

II

worin

$R^1$ und $R^4$ die in Anspruch 1 angegebenen Bedeutungen haben und

∿∿∿ anzeigt, dass diese Bindung α- oder β-ständig sein kann mit einer Verbindung der Formel III

$$MS–CH_2–CR^5(OR^3)–D–R^2 \qquad III$$

worin

M H oder ein Äquivalent eines Kations bedeutet und

D, $R^2$, $R^3$ und $R^5$ die in Anspruch 1 angegebenen Bedeutungen haben

umsetzt

und/oder dass man gegebenenfalls eine Verbindung der Formel I, worin $R^1$ und/oder $R^3$ und/

oder $R^4$ von H verschieden ist (sind), durch Behandeln mit solvolysierenden Mitteln in eine Verbindung der Formel I, worin $R^1$ und/oder $R^3$ und/oder $R^4$ H ist (sind), überführt und/oder eine Verbindung der Formel I, worin $R^1$=H ist, verestert und/oder eine Verbindung der Formel I, worin $R^3$ und/oder $R^4$ H ist (sind), verethert oder verestert und/oder eine als Racemat erhaltene Verbindung der Formel I in ihre Enantiomeren aufspaltet und/oder eine Verbindung der Formel I, worin $R^1$=H ist, durch Umsetzen mit einer Base in eines ihrer Salze überführt.

4. Verfahren zur Herstellung pharmazeutischer Zubereitungen, dadurch gekennzeichnet, dass man mindestens eine Verbindung der allgemeinen Formel I und/oder eines ihrer physiologisch unbedenklichen Metall- oder Ammoniumsalze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Metall- oder Ammoniumsalze.

6. Verbindungen der allgemeinen Formel I zur Bekämpfung von Krankheiten.

7. Verwendung von Verbindungen der allgemeinen Formel I zur Herstellung pharmazeutischer Zubereitungen.

## Revendications

1. 6-cétoprostaglandines renfermant du soufre, de formule I:

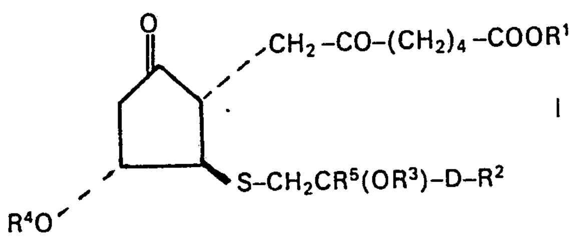

I

dans laquelle

D représente une laision, un alcoylène ayant 1 à 3 atomes de carbone, un cis-alcénylène ayant 2 à 5 atomes de carbone ou un alcinylène ayant 2 à 5 atomes de carbone,

$R^1$ H, alcoyle ayant 1 à 4 atomes de carbone, aryle ayant 6 à 12 atomes de carbone ou $-C_6H_4NHCOC_6H_4$

$R^2$ alcoyle ayant 1 à 7 atomes de carbone, alcoyle ayant 1 à 7 atomes de carbone et substitué par de l'halogène, cycloalcoyle ayant 5 à 6 atomes de carbone, cycloalcoyle ayant 5 à 6 atomes de carbone et substitué par un alcoyle ayant 1 à 4 atomes de carbone, phényle, phényle substitué par F, Cl, Br, alcoyle ayant 1 à 4 atomes de carbone, OH, $OCH_3$ ou $CF_3$, pyridyle, naphthyle, thiényle, ou bien lorsque D est un alcoylène ayant 1 à 3 atomes de carbone, également un alcoxy ayant 1 à 4 atomes de carbone, alcoylthio ayant 1 à 4 atomes de carbone, phénoxy ou phénoxy substitué par F, Cl, Br, alcoyle ayant 1 à 4 atomes de carbone, OH, $OCH_3$ ou $CF_3$,

$R^3$ et $R^4$ sont chacun H, alcoyle ayant 1 à 7 atomes de carbone, tétrahydropyrane-2-yle, trialcoylsilyle avec en tout 3 à 12 atomes de carbone, aryl-dialcoysilyle ayant en tout 8 à 18 atomes de carbone, alcoxyméthyle ayant 2 à 5 atomes de carbone, aryloxyméthyle ayant 7 à 11 atomes de carbone ou acyle ayant 1 à 10 atomes de carbone et

$R^5$ est un H ou un alcoyle ayant 1 à 3 atomes de carbone, tandis que

....... indique que cette liaison est en position α,

▬ indique que cette liaison est en position β,

de même que, lorsque $R^1$=H, leurs sels.

2. a) 6,9-dioxo-11α-t-butyldiméthylsilyloxy-13-thia-16,20-diméthyl-prostanoate de p-benzamidophényle

b) 6,9-dioxo-11α-t-butyldiméthylsilyloxy-13-thia-15-hydroxy-15-méthyl-prostanoate de p-benzamidophényle

c) 6,9-dioxo-11α,15-dihydroxy-13-thia-15-méthyl-prostanoate de p-benzamidophényle.

3. Procédé de fabrication de 6-cétoprostaglandines renfermant du soufre de formule I selon la revendication 1, de même que, lorsque $R^1$=H, de leurs sels, caractérisé en ce qu'on fait réagir un composé de formule II:

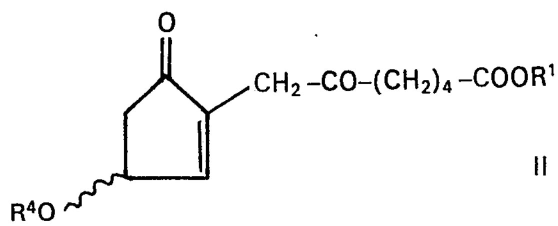

II

dans laquelle

$R^1$ et $R^2$ ont les significations indiqué es à la revendication 1 et

∿∿∿ indique que cette liaison peut être en position α ou β, avec un composé de formule III

$$MS-CH_2-CR^5(OR^3)-D-R^2 \qquad III$$

dans laquelle

M signifie H ou un équivalent d'un cation et

D, $R^2$, $R^3$ et $R^5$ ont les significations indiquées à la revendication 1, et/ou en ce qu'on convertit éventuellement un composé de formuel I dans laquelle $R^1$ et/ou $R^3$ et/ou $R^4$ est (ou sont) différent(s) de H, par traitement avec des agents de solvolyse, en un composé de formule I dans laquelle $R^1$ et/ou $R^3$ et/ou $R^4$ est (ou sont) H, et/ou en ce qu'on estérifie un composé de formule I dans laquelle $R^1$=H, et/ou on ethérifie ou estérifie un composé de formule I dans laquelle $R^3$ et/ou $R^4$ est (ou sont) H, et/ou en ce qu'on clive un composé de formule I obtenu à l'état de racémate en ses énantiomères, et/ou en ce qu'on convertit un composé de formuel I dans laquelle

$R^1$=H, en ses sels par réaction avec une base.

4. Procédé de fabrication de préparations pharmaceutiques, caractérisé en ce qu'on met sous une forme dosée appropriée au moins un composé de formule générale I et/ou un de ses sels métalliques ou d'ammonium physiologiquement inoffensifs conjointement avec au moins une matière de support solide, liquide ou semi-liquide ou une substance auxiliaire et éventuellement en combinaison avec une ou plusieurs autre(s) matière(s) active(s).

5. Préparation pharmaceutique, caractérisée par une teneur en au moins un composé de formuel générale I et/ou en un de ses sels métalliques ou d'ammonium physiologiquement inoffensifs.

6. Composés selon la formule générale I pour combattre les maladies.

7. Utilisation de composés de formule générale I pour la fabrication de préparations pharmaceutiques.

## Claims

1. Sulfur-containing 6-ketoprostaglandins of the formula I

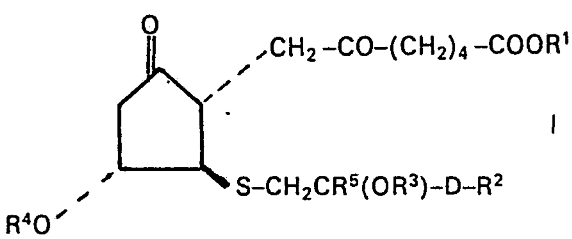

I

in which

D is a bond, alkylene having 1–3 C atoms, cis-alkenylene having 2–5 C atoms or alkinylene having 2–5 C atoms,

$R^1$ is H, alkyl having 1–4 C atoms, aryl having 6–12 C atoms or $-C_6H_4NHCOC_6H_5$,

$R^2$ is alkyl having 1–7 C atoms, alkyl having 1–7 C atoms which is substituted by halogen, cycloalkyl having 5–6 C atoms, cycloalkyl having 5–6 C atoms which is substituted by alkyl having 1–4 C atoms, phenyl, phenyl which is substituted by F, Cl, Br, alkyl having 1–4 C atoms, OH, $OCH_3$ or $CF_3$, pyridyl, naphthyl, thienyl or, if D is alkylene having 1–3 C atoms, also is alkoxy having 1–4 C atoms, alkylthio having 1–4 C atoms, phenoxy or phenoxy which is substituted by F, Cl, Br, alkyl having 1–4 C atoms, OH, $OCH_3$ or $CF_3$,

$R^3$ and $R^4$ each are H, alkyl having 1–7 C atoms, tetrahydro-2-pyranyl, trialkylsilyl having a total of 3–12 C atoms, aryldialkylsilyl having a total of 8–18 C atoms, alkoxymethyl having 2–5 C atoms, aryloxymethyl having 7–11 C atoms or acyl having 1–10 C atoms, and

$R^5$ is H or alkyl having 1–3 C atoms, and

....... indicates that this bond is α,

▬▬ indicates that this bond is β,

and, where $R^1$ is H, their salts.

2. a) p-Benzamidophenyl 6,9-dioxo-11α-tert.-butyl dimethylsilyloxy-13-thia-16,20-dimethyl-prostanoate;

b) p-Benzamidophenyl 6,9-dioxo-11α-tert.-butyl-dimethylsilyloxy-13-thia-15- hydroxy-15-methyl-prostanoate;

c) p-Benzamidophenyl 6,9-dioxo-11α,15-dihydroxy- 13-thia-15-methylprostanoate.

3. Process for the preparation of sulfur-containing 6-ketoprostaglandins of the formula I according to claim 1, and, where $R^1$ is H, of their salts, characterised in that a compound of the formula II

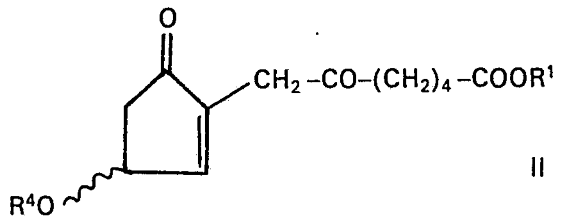

II

in which

$R^1$ and $R^4$ have the meanings indicated in claim 1, and

∿∿ indicates that this bond can be α or β, is reacted with a compound of the formula III

$MS-CH_2-CR^5(OR^3)-D-R^2$ III

in which

M is H or one equivalent of a cation, and

D, $R^2$, $R^3$ and $R^5$ have the meanings indicated in claim 1, and/or in that, where appropriate, a compound of the formula I, in which $R^1$ and/or $R^3$ and/or $R^4$ differ(s) from H, is converted, by treatment with solvolysing agents, into a compound of the formula I, in which $R^1$ and/or $R^3$ and/or $R^4$ is (are) H, and/or a compound of the formula I, in which $R^1$ is H, is esterified and/or a compound of the formula I, in which $R^3$ and/or $R^4$ is (are) H, is etherified or esterified and/or a compound of the formula I which is obtained as a racemate is resolved into its enantiomers and/or a compound of the formula I, in which $R^1$ is H, is converted into one of its salts by reaction with a base.

4. Process for the preparation of pharmaceutical formulations, characterised in that at least one compound of the general formula I and/or one of its physiologically acceptable metal or ammonium salts is converted into a suitable form for administration, together with at least one solid, liquid or semi-liquid vehicle or auxiliary and, where appropriate, in combination with one or more other active compound(s).

5. Pharmaceutical formulation characterised by containing at least one compound of the general formula I and/or one of its physiologically acceptable metal or ammonium salts.

6. Compounds of the general formula I for combating diseases.

7. Use of compounds of the general formula I for the preparation of pharmaceutical formulations.